# EUROPÄISCHE PATENTSCHRIFT

(11) **EP 1 305 281 B1**
(45) Veröffentlichungstag und Bekanntmachung des Hinweises auf die Patenterteilung: **07.04.2004**
(21) Anmeldenummer: 01951675.6
(22) Anmeldetag: 13.07.2001
(51) Int. Cl.: C07C 311/13, C07C 227/04, C07C 229/38, C07C 303/38, C07C 303/40, C07C 311/42, C07D 239/52

(54) **SUBSTITUIERTE SULFONYLAMINOMETHYLBENZOESÄURE(DERIVATE) UND VERFAHREN ZU IHRER HERSTELLUNG**
SUBSTITUTED SULFONYL AMINOMETHYL BENZOIC ACID (DERIVATIVES) AND METHOD FOR THE PRODUCTION THEREOF
ACIDE SULFONYLAMINOMETHYLBENZOIQUE ET DERIVES, ET PROCEDE DE FABRICATION

(30) Priorität: 24.07.2000 DE 10036184
(43) Veröffentlichungstag der Anmeldung: 02.05.2003
(73) Patentinhaber: Bayer CropScience GmbH, 65929 Frankfurt (DE)
(72) Erfinder: LORENZ, Klaus, 64331 Weiterstadt (DE); RESSEL, Hans-Joachim, 65795 Hattersheim (DE); WILLMS, Lothar, 65719 Hofheim (DE)
(86) Internationale Anmeldenummer: PCT/EP2001/008111
(87) Internationale Veröffentlichungsnummer: WO 2002/008176

(56) Entgegenhaltungen:
- DE-A- 4 335 297
- W.S. SAARI, ET AL.: "A convenient synthesis of nitro-substituted 1,2-benzothiazol-3(2H)-one 1,1-dioxides (nitrosaccharins)" JOURNAL OF HETEROCYCLIC CHEMISTRY, Bd. 23, Nr. 4, Juli 1986 (1986-07), Seiten 1253-1255, XP002178946 Heterocorporation, Provo, US ISSN: 0022-152X

## Beschreibung

Die Erfindung betrifft das technische Gebiet der Zwischenprodukte für die Herstellung von Wirkstoffen, insbesondere von herbizid wirksamen Sulfonylharnstoffen.

Es ist bekannt, daß man aromatische Amine zu Sulfonsäurederivaten wie Sulfochloriden und weiter zu Sulfonamiden umsetzen kann, weiche ihrerseits zur Herstellung von herbizid wirksamen Sulfonylharnstoffen eingesetzt werden können (Meerwein et al., Chem. Berichte 90, 841-852 (1957) und EP-A-574418).

Aus J. Med. Chem. 1986, Vol. 29, No. 4, Seite 585 ist eine substituierte Anthranilsäure als Zwischenprodukt zur Herstellung von bestimmten Anhydriden bekannt, die sich zur Inaktivierung trypsinartiger Enzyme eignen.

Es bestand die Aufgabe, neue chemische Verbindungen zur Verfügung zu stellen, welche sich zur Herstellung herbizid wirksamer Sulfonylharnstoffe eignen. Diese Aufgabe wird überraschenderweise gelöst durch Verbindungen der Formel (I), 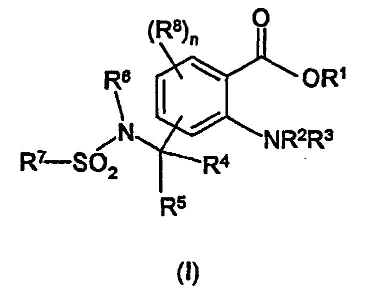 worin
- R¹: H, (C₁-C₈)Alkyl, (C₃-C₈)Alkenyl oder (C₃-C₈)Alkinyl ist, wobei die letzten 3 Reste unsubstituiert oder substituiert sind, z.B. durch einen oder mehrere Reste aus der Gruppe Halogen, (C₁-C₄)Alkoxy, (C₁-C₄)Alkylthio, [(C₁-C₄)Alkyl]carbonyl oder [(C₁-C₄)Alkoxy]carbonyl,
- R², R³: unabhängig voneinander H oder Acyl sind, vorzugsweise H sind,
- R⁴, R⁵: H sind,
- R⁶: H oder (C₁-C₈)Alkyl ist, welches unsubstituiert oder substituiert ist, z.B. durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, [(C₁-C₄)Alkyl]carbonyl oder CN, vorzugsweise H ist,
- R⁷: (C₁-C₈)Alkyl, (C₃-C₈)Alkenyl oder (C₃-C₈)Alkinyl ist, welche unsubstituiert oder substituiert sind, z. B. durch einen oder mehrere Reste aus der Gruppe Halogen, (C₁-C₄)Alkoxy oder (C₁-C₄)Alkylthio, oder R⁷ ist (C₆-C₁₄)-Aryl (z.B. Phenyl), welches unsubstituiert oder substituiert ist, z.B. durch einen oder mehrere Reste aus der Gruppe Halogen, NO₂, CN, (C₁-C₄)Alkyl, (C₁-C₄)Haloalkyl oder (C₁-C₄)Alkoxy, oder R⁷ ist Mono- oder Di-(C₁-C₈)Alkylamino, welches unsubstituiert oder substituiert ist, z.B. durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, [(C₁-C₄)Alkyl]carbonyl, [(C₁-C₄)Alkoxy]carbonyl oder CN, oder
- R⁶ und R⁷: gemeinsam eine Kette der Formel -(CH₂)ₘBₘ₁-bilden, welche unsubstituiert oder substituiert ist, z.B. durch einen oder mehrere (C₁-C₄)Alkylreste, und worin m=2, 3 oder 4, m¹=0 oder 1 und B=CO oder SO₂ bedeutet,
- R⁸: gleich oder verschieden (C₁-C₄)Alkyl, (C₁-C₄)Alkoxy, [(C₁-C₄)Alkyl]carbonyl oder [(C₁-C₄)Alkoxy]carbonyl sind, welche unsubstituiert oder substituiert sind, z.B. durch einen oder mehrere Reste aus der Gruppe Halogen, (C₁-C₄)Alkoxy, (C₁-C₄)Alkylthlo, [(C₁-C₄)Alkyl]carbonyl oder [(C₁-C₄)Alkoxy]carbonyl, oder R⁸ ist Halogen oder NH₂, und
- n: ist gleich 0, 1, 2 oder 3, vorzugsweise gleich 0 ist.

Bevorzugt sind Verbindungen der Formel (I), worin
- R¹: H oder (C₁-C₄)-Alkyl, vorzugsweise (C₁-C₄)Alkyl ist,
- R² und R³: H sind,
- R⁴ und R⁵: H sind,
- R⁶: H ist,
- R⁷: (C₁-C₄)Alkyl ist, und
- n: gleich 0 ist.

Von besonderer Bedeutung sind Verbindungen der Formel (I), worin die Gruppe CR⁴R⁵-NR⁶-SO₂-R⁷ in para-Stellung zur Gruppe -CO-OR¹ steht. Wenn R⁶ und R⁷ gemeinsam eine Kette der Formel -(CH₂)ₘBₘ₁- bilden und m¹=1 ist, ist es bevorzugt, wenn B an das R⁶-tragende Stickstoffatom gebunden ist.

Beispiele für Verbindungen der Formel (I) sind in der nachfolgenden Tabelle 1 aufgeführt:

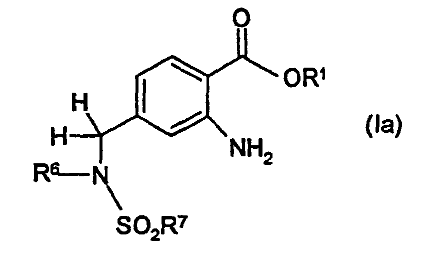

**Tabelle 1**

| Verbindung | R¹ | R⁶ | R⁷ |
|---|---|---|---|
| 1 | Me | H | Me |
| 2 | Me | Me | Me |
| 3 | Me | H | NHMe |
| 4 | Me | Me | NHMe |
| 5 | Me | H | N(Me)₂ |
| 6 | Me | Me | N(Me)₂ |
| 7 | Me | -CH₂-CH₂-CH₂- | |
| 8 | Me | -CH₂-CH₂-CH₂-CH₂- | |
| 9 | Me | H | Phe |
| 10 | Me | Me | Phe |
| 11 | Me | H | CH₂F |
| 12 | Me | Me | CH₂F |
| 13 | Me | H | CF₃ |
| 14 | Me | Me | CF₃ |
| 15 | Me | H | Et |
| 16 | Me | Me | Et |
| 17 | Me | H | nPr |
| 18 | Me | Me | nPr |
| 19 | Me | H | iPr |
| 20 | Me | Me | iPr |
| 21 | Me | H | nBu |
| 22 | Me | Me | nBu |
| 23 | Me | Et | Me |
| 24 | Me | Et | Et |
| 25 | Me | Et | NHMe |
| 26 | Me | Et | N(Me)₂ |
| 27 | Me | Et | Phe |
| 28 | Me | Et | CH₂F |
| 29 | Me | Et | CF₃ |
| 30 | Me | Et | nPr |
| 31 | Me | Et | iPr |
| 32 | Me | Et | nBu |
| 33 | Et | H | Me |
| 34 | Et | Me | Me |
| 35 | Et | H | NHMe |
| 36 | Et | Me | NHMe |
| 37 | Et | H | N(Me)₂ |
| 38 | Et | Me | N(Me)₂ |
| 39 | Et | -CH₂-CH₂-CH₂- | |
| 40 | Et | -CH₂-CH₂-CH₂-CH₂- | |
| 41 | Et | H | Phe |
| 42 | Et | Me | Phe |
| 43 | Et | H | CH₂F |
| 44 | Et | Me | CH₂F |
| 45 | Et | H | CF₃ |
| 46 | Et | Me | CF₃ |
| 47 | Et | H | Et |
| 48 | Et | Me | Et |
| 49 | Et | H | nPr |
| 50 | Et | Me | nPr |
| 51 | Et | H | iPr |
| 52 | Et | Me | iPr |
| 53 | Et | H | nBu |
| 54 | Et | Me | nBu |
| 55 | Et | Et | Me |
| 56 | Et | Et | Et |
| 57 | Et | Et | NHMe |
| 58 | Et | Et | N(Me)₂ |
| 59 | Et | Et | Phe |
| 60 | Et | Et | CH₂F |
| 61 | Et | Et | CF₃ |
| 62 | Et | Et | nPr |
| 63 | Et | Et | iPr |
| 64 | Et | Et | nBu |
| In der Tabelle 1 bedeuten Me = Methyl, Et = Ethyl, nPr = n-Propyl, iPr = iso-Propyl, nBu = n-Butyl, Phe = Phenyl. | | | |

Soweit in dieser Beschreibung der Begriff Acyl verwendet wird bedeutet dieser den Rest einer organischen Säure, der formal durch Abspaltung einer OH-Gruppe aus der organischen Säure entsteht, z.B. der Rest einer Carbonsäure und Reste davon abgeleiteter Säuren wie der Thiocarbonsäure, gegebenenfalls N-substituierten Iminocarbonsäuren oder die Reste von Kohlensäuremonoestem, gegebenenfalls N-substituierter Carbaminsäuren, Sulfonsäuren, Sulfinsäuren, Phosphonsäuren, Phosphinsäuren.

Ein Acylrest ist bevorzugt Formyl oder Acyl aus der Gruppe CO-R^{x}, CS-R^{x}, CO-OR^{x}, CS-OR^{x}, CS-SR^{x}, CR^{x}=NR^{Y}, SOR^{Y} oder SO₂R^{Y}, wobei R^{x} und R^{Y} jeweils einen C₁-C₁₀-Kohlenwasserstoffrest wie C₁-C₁₀-Alkyl oder C₆-C₁₀-Aryl bedeuten, der unsubstituiert oder substituiert ist, z.B. durch einen oder mehrere Substituenten aus der Gruppe Halogen wie F, Cl, Br, I, Alkoxy, Haloalkoxy, Hydroxy, Amino, Nitro, Cyano oder Alkylthio, oder Acyl bedeutet Aminocarbonyl oder Aminosulfonyl, wobei die beiden letztgenannten Reste unsubstituiert, N-monosubstituiert oder N,N-disubstituiert sind, z.B. durch Substituenten aus der Gruppe Alkyl oder Aryl.
Acyl bedeutet beispielsweise Formyl, Halogenalkylcarbonyl, Alkylcarbonyl wie (C₁-C₄)Alkylcarbonyl, Phenylcarbonyl, wobei der Phenylring substituiert sein kann, oder Alkyloxycarbonyl, wie (C₁-C₄) Alkyfoxycarbonyl, Phenyloxycarbonyl, Benzyloxycarbonyl, Alkylsulfonyl, wie (C₁-C₄) Alkylsulfonyl, Alkylsulfinyl, wie C₁-C₄(Alkylsulfinyl), N-Alkyl-1-iminoalkyl, wie N-(C₁-C₄)-1-imino-(C₁-C₄)alkyl und andere Reste von organischen Säuren.

In der Formel (I) und den im folgenden verwendeten allgemeinen Formeln können die Reste Alkyl, Alkoxy, Haloalkyl, Haloalkoxy und Alkylthio sowie die entsprechenden substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 4 C-Atomen bevorzugt. Alkylreste, auch in den zusammengesetzten Bedeutungen wie Alkoxy, Haloalkyl usw. bedeuten, z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl z.B. in der Form "(C₃-C₈)Alkenyl" bedeutet vorzugsweise einen Alkenylrest mit 3 bis 8 C-Atomen, bei dem die Doppelbindung nicht an dem C-Atom liegt, das mit dem übrigen Molekülteil der Verbindung (I) verbunden ist ("yl"-Position). Entsprechendes gilt für Alkinylreste.

Halogen bedeutet beispielsweise Fluor, Chlor, Brom oder Iod. Haloalkyl, -alkenyl und - alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl₂, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyloxy und andere durch Halogen substituierte Reste.

Substituierte Reste, wie substituierte Kohlenwasserstoffreste, z.B. substituiertes Alkyl, Alkenyl, Alkinyl, Aryl, z.B. Phenyl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohfenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Gegebenenfalls substituiertes. Phenyl oder Phenoxy ist vorzugsweise Phenyl oder Phenoxy, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und Trichlorphenyl, 2,4-, 3,5-, 2,5- und 2,3-Dichlorphenyl, o-, m- und p-Methoxyphenyl.

Sind Substitutionen definiert durch einen oder mehrere Reste aus einer Gruppe von Resten beinhaltet dies sowohl die Substitution durch einen oder mehrere gleiche Reste als auch die einfache oder mehrfache Substitution durch unterschiedliche Reste.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome, die in der allgemeinen Formel (I) nicht gesondert angegeben sind. Die durch ihre spezifische Raumform definierten möglichen Stereoisomeren, wie Enantiomere, Diastereomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden. Formel (I) umfaßt auch Tautomere der bezeichneten Verbindungen, soweit sie durch Protonenwanderung entstehen und soweit sie chemisch stabil sind.

Die Verbindungen der Formel (I) können Salze bilden, bei denen ein acides Wasserstoffatom durch ein geeignetes Kation ersetzt ist. Diese Salze sind beispielsweise Metallsalze; vorzugsweise Alkali- oder Erdalkalisalze, insbesondere Natrium- und Kaliumsalze, oder auch Ammoniumsalze oder Salze mit organischen Aminen. Ebenso kann Salzbildung durch Anlagerung einer Säure an basische Gruppen, wie Amino, erfolgen. Geeignete Säuren hierfür sind starke anorganische und organische Säuren, beispielsweise HCl, HBr, H₂SO₄, HNO₃ oder Ameisensäure.

Die Synthese von Verbindungen der Formel (I) gelingt ausgehend von Verbindungen der nachfolgend genannten Formel (II) in sehr guten Ausbeuten und Reinheiten.

Gegenstand der vorliegenden Erfindung ist somit auch ein Verfahren zur Herstellung von Verbindungen der Formel (I), enthaltend die Schritte, worin
1a) eine Verbindung der Formel (II) 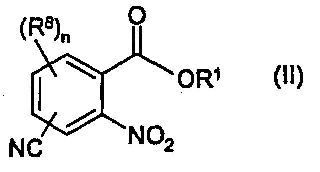 durch katalytische Hydrierung in Abwesenheit einer Säure zu einer Verbindung der formel (III) oder durch katalytische Hydrierung in Gegenwart einer Säure, z.B. H⁺X⁻, worin X⁻ ein Äquivalent eines Säureanions ist, wie Halogen z.B. Cl⁻, Br⁻ oder I⁻, oder HSO₄⁻, ½ SO₄²⁻, H₂PO₄⁻, ½ HPO₄²⁻, ¹/₃ PO₄³⁻ oder ⁻OCOR (wobei R = H oder (C₁-C₈)Alkyl bedeutet), zu einer Verbindung der Formel (IIIa), worin X⁻ ein Äquivalent eines Säureanions ist, wie Halogenid z.B. Cl⁻, Br⁻ oder I⁻, oder HSO₄⁻, ½ SO₄²⁻, H₂PO₄⁻, ½ HPO₄²⁻, ¹/₃ PO₄³⁻ oder ⁻OCOR (wobei R = H oder (C₁-C₈)Alkyl bedeutet), umgesetzt wird, 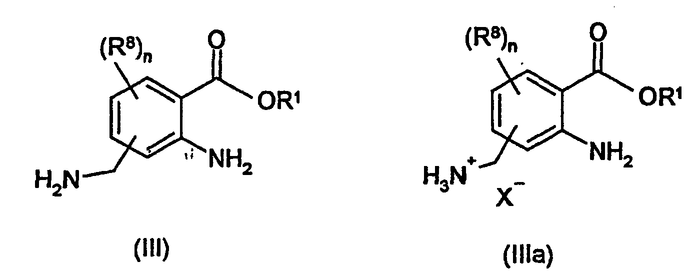 und anschließend
1b) die Verbindung der Formel (III) oder (IIIa) mit einem Sulfonsäurederivat zu einer Verbindung der Formel (I) mit R², R³ und R⁶=H umgesetzt wird; oder
2a)
   α) eine Verbindung der Formel (II) 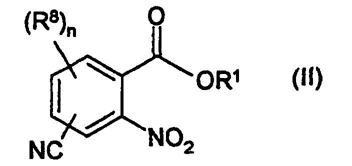 durch gängige Reduktionsverfahren für Nitroverbindungen zu einer Verbindung der Formel (IV) umgesetzt wird, 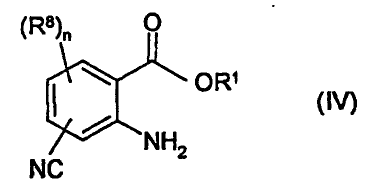 und anschließend
   β) die Verbindung der Formel (IV) entweder durch katalytische Hydrierung oder durch gängige Reduktionsverfahren für Nitrile, zu einer Verbindung der Formel (III) oder (IIIa) umgesetzt wird,
   und anschließend
2b) die Verbindung der Formel (III) oder (IIIa) mit einem Sulfonsäurederivat zu einer Verbindung der Formel (I) mit R², R³ und R⁶=H umgesetzt wird; oder
3a)
   α) eine Verbindung der Formel (II) 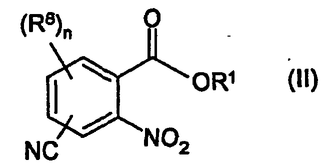 durch gängige Reduktionsverfahren für Nitrile zu einer Verbindung der Formel (V) oder (Va), worin X^{e} wie in Formel (IIIa) definiert ist, umgesetzt wird, 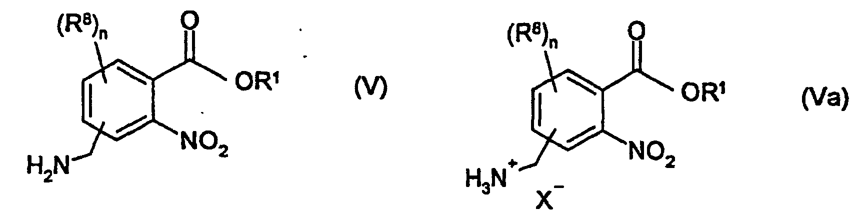 und anschließend
   β) die Verbindung der Formel (V) oder (Va) durch gängige Reduktionsverfahren für Nitroverbindungen oder durch katalytische Hydrierung zu einer Verbindung der Formel (III) oder (IIIa) umgesetzt wird,
   und anschließend
3b) die Verbindung der Formel (III) oder (IIIa) mit einem Sulfonsäurederivat zu einer Verbindung der Formel (I) mit R², R³ und R⁶=H umgesetzt wird; oder
4a)
   α) eine Verbindung der Formel (II) 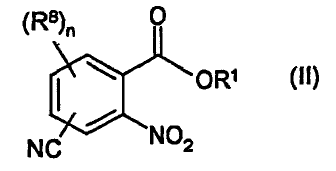 durch gängige Reduktionsverfahren für Nitrile zu einer Verbindung der Formel (V) oder (Va), worin X⁻ wie in Formel (IIIa) definiert ist, umgesetzt wird, 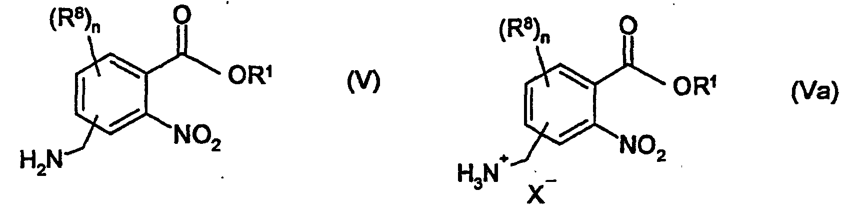
   β) und anschließend die Verbindung der Formel (V) oder (Va) mit einem Sulfonsäurederivat zu einer Verbindung der Formel (VI) umgesetzt wird, 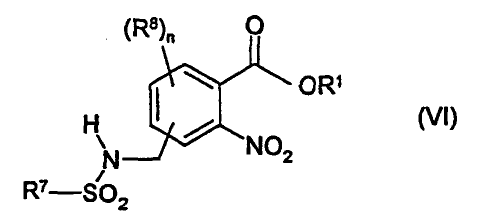
   und anschließend
4b) die Verbindung der Formel (VI) durch gängige Reduktionsverfahren für Nitroverbindungen oder durch katalytische Hydrierung zu einer Verbindung der Formel (I) mit R², R³ und R⁶=H umgesetzt wird.

Die Verbindungen der Formeln (III), (IIIa), (V), (Va) und (VI) sind neu und ebenfalls Gegenstand der vorliegenden Erfindung.

Verbindungen der Formel (I) mit R² und/oder R³ = Acyl können erhalten werden, indem Verbindungen der Formel (I) mit R² und R³ = H mit Acylierungsmitteln wie Carbonsäure-, Sulfonsäure- und Carbamoylhalogeniden, Carbonsäure- und Sulfonsäureanhydriden, Halogenameisensäureestern oder Isoyanaten nach gängigen Methoden acyliert werden (siehe z.B. L.-F. Tietze, Th. Eicher, Reaktionen und Synthesen im organisch-chemischen Praktikum, Thieme Verlag Stuttgart/New York, 1981, S. 131, 316, 318, 345; R.C. Larock, Comprehensive Organic Transformations (1989), S. 979, 981). Als Lösungsmittel eignen sich z.B. aprotische Lösungsmittel wie Dichlormethan, Acetonitril, Dioxan, Tetrahydrofuran, Toluol oder Chlorbenzol, vorzugsweise bei Temperaturen von 0°C bis zum Siedepunkt des Lösungsmittels.

Verbindungen der Formel (I) mit R⁶ = unsubstituiertes oder substituiertes C₁-C₈-Alkyl können z.B. erhalten werden, indem Verbindungen der Formel (I) mit R⁶ = H mit Alkylierungsmitteln wie Alkylhalogeniden, Alkylsulfaten wie Dimethylsulfat oder Alkyltosylaten nach gängigen Methoden alkyliert werden. Als Lösungsmittel eignen sich z.B. Aceton und Dimethylformamid (vgl. z.B. R.C. Larock, Comprehensive Organic Transformations (1989), S. 398; L-F. Tietze, Th. Eicher, Reaktionen und Synthesen im organisch-chemischen Praktikum, Thieme Verlag Stuttgart/New York, 1981, S. 75; Organikum, Organisch-chemisches Grundpraktikum VEB, Berlin 1981). Die Alkylierung kann in Gegenwart von Basen wie K₂CO₃, NaH oder Alkoholaten wie Na-Alkoholat durchgeführt werden. Vorzugsweise wird dabei von Verbindungen der Formel (I) ausgegangen, worin R² und R³ gleich Acyl sind.

Verbindungen der Formel (I) mit R⁶ = unsubstituiertes oder substituiertes C₁-C₈-Alkyl können z.B. auch über reduktive Aminierungen z.B. mit Aldehyden oder Ketonen in Gegenwart von Reduktionsmitteln wie H₂/Katalysator, Ameisensäure, Zink/HCl, Natriumborhydrid oder Natriumcyanoborhydrid erhalten werden. Ein Beispiel ist die Leuckard-Wallach-Reaktion mit Formaldehyd und Ameisensäure.

Bevorzugt sind Verfahren, bei denen die Nitro- und die Nitrilgruppe in Verbindungen der Formel (II) in einem Verfahrensschritt gemeinsam mittels katalytischer Hydrierung entsprechend Verfahrensvariante 1a) zu Verbindungen der Formel (III) oder (IIIa) reduziert werden.

Die in dem erfindungsgemäßen Verfahren als Zwischenverbindungen erhaltenen Aminoverbindungen der Formeln (III) und (V) können auch in Form ihrer Salze (IIIa) und (Va) entstehen und weiter umgesetzt werden, wenn die Umsetzung oder die Aufarbeitung in einem sauren Medium geschieht.

Die in den Formeln (II), (III), (IIIa), (IV), (V), (Va) und (VI) angegebenen Symbole haben die gleiche Bedeutung wie in Formel (I), einschließlich der hierfür angegebenen Vorzugsbereiche. Bevorzugt sind Verbindungen der Formeln (II), (III), (IIIa), (IV), (V), (Va) und (VI), worin die Gruppen -CN (Formeln (II) und (IV)), -CH₂-NH₂ (Formeln (III) und (V)), -CH₂-NH₃⁺X⁻ (Formeln (IIIa) und (Va)) bzw. -CH₂-NR⁶-SO₂-R⁷ (Formel (VI)) in para-Stellung zur Gruppe -CO-OR¹ stehen.

Weiterhin sind Gegenstand der Erfindung auch Teilschritte des erfindungsgemäßen Verfahrens.

Die Verbindungen der Formel (II) sind bekannt, vgl. z.B. DE 22 39 799 C3 oder Journal of the American Chemical Society 99, 6721 (1977).

Die katalytische Hydrierung der Verbindung der Formel (II) nach Verfahrensvariante 1a), der Verbindung der Formel (IV) nach Verfahrensvariante 2aβ), der Verbindung der Formel (V) oder (Va) nach Verfahrensvariante 3aβ) oder der Verbindung der Formel (VI) nach Verfahrensvariante 4b) gelingt mittels gängiger Hydrierverfahren. Als Wasserstoffquelle können z.B. Wasserstoff-Gas, Hydrazin oder HN=NH verwendet werden. Als Hydrierkatalysatoren sind insbesondere Edelmetallkatalysatoren, z.B. Pd, Pt, Rh, Ir, oder Ni- oder Co-Katalysatoren geeignet. Die Edelmetalle können dabei in elementarer Form oder in Form von Oxiden oder Halogeniden verwendet werden. Die Edelmetallkatalysatoren können wahlweise ohne oder vorzugsweise mit Trägermaterialien wie Aktivkohle, Kieselgur, Silikate verwendet werden.

Die Hydrierung kann sowohl bei Atmosphärendruck als auch durch Anwendung eines Wasserstoffüberdrucks, in der Regel zwischen 1 und 100 bar, vorzugsweise 1-50 bar durchgeführt werden. Die geeignete Temperatur liegt im allgemeinen im Bereich von -20 bis 150°C, vorzugsweise zwischen 0 und 120°C.

Für die Hydrierung geeignete Lösungsmittel sind z.B. Lösungsmittel aus den Gruppen Wasser, Alkohole wie Methanol oder Ethanol, Ether wie Diethylether, Tetrahydrofuran oder Dioxan, Amide wie Dimethylformamid oder Dimethylacetamid, Ester wie Essigsäureethylester, organische Carbonsäuren wie Ameisensäure oder Essigsäure, aromatische Kohlenwasserstoffe wie Toluol, Xylol oder Chlorbenzol, oder halogenierte aliphatische Kohlenwasserstoffe wie CH₂Cl₂, wobei die Lösungsmittel in reiner Form oder als Gemische eingesetzt werden können.

Die katalytische Hydrierung der Verbindungen der Formel (II) nach Verfahrensvariante 1a) oder der Verbindungen der Formel (IV) nach Verfahrensvariante 2aβ) wird vorzugsweise in Gegenwart von 1-10 Moläquivalenten einer Säure durchgeführt. Als Lösungsmittel werden bevorzugt Alkohole wie Methanol oder Ethanol, oder Wasser verwendet. Geeignete Säuren sind z.B. Mineralsäuren oder Carbonsäuren. Bevorzugt sind Säuren H⁺X⁻, worin X⁻ ein Äquivalent eines Säurerests ist, wie Halogen z.B. Cl⁻, Br⁻ oder I⁻, oder HSO₄⁻, ½ SO₄²⁻, H₂PO₄⁻, ½ HPO₄²⁻, ¹/₃ PO₄³⁻ oder ⁻OCOR (wobei R = H oder (C₁-C₈)Alkyl bedeutet), z.B. Halogenwasserstoffsäuren wie Salzsäure oder Bromwasserstoffsäure, oder Schwefelsäure, Phosphorsäure, Ameisensäure oder Essigsäure. Die Säuren können z.B. bei Verwendung der beiden letztgenannten Säuren auch komplett die Rolle des Lösungsmittels einnehmen.

Die katalytische Hydrierung der Verbindungen der Formel (IV) kann auch durch Verwendung von 1-10 Moläquivalenten Ammoniak durchgeführt werden, wobei vorzugsweise Ni- oder Co-Katalysatoren wie Raney-Nickel oder Raney-Cobalt eingesetzt werden. Als Lösungsmittel werden hierbei bevorzugt Alkohole wie Methanol oder Ethanol verwendet.

Die Reduktion der Nitrogruppe in Verbindungen der Formel (II) nach Verfahrensvariante 2aα), Verbindungen der Formel (V) und (Va) nach Verfahrensvariante 3aβ) oder Verbindungen der Formel (VI) nach Verfahrensvariante 4b) kann mit gängigen Reduktionsmitteln für aromatische Nitroverbindungen erfolgen. Solche Reduktionsmittel und Reaktionsbedingungen sind z.B. beschrieben in R.C. Larock, Comprehensive Organic Transformations (1989) S. 411-415, VCH Publishers Inc. und der dort angegebenen Literatur. Bevorzugte Reduktionsmittel sind z.B. Fe, Zn, Sn oder deren Salze wie FeSO₄ oder Sn-II-Salze wie SnCl₂. Als Lösungsmittel eignen sich z.B. organische Carbonsäuren, Alkohole und/oder Mineralsäuren. Die Reaktionstemperatur liegt im allgemeinen zwischen 0°C und dem Siedepunkt des Lösungsmittels.

Die Reduktion der Nitrilgruppe in Verbindungen der Formel (IV) nach Verfahrensvariante 2aβ) und Verbindungen der Formel (II) nach Verfahrensvariante 3aα) und 4aα) kann durch gängige Reduktionsmittel für Nitrile erfolgen. Solche Reduktionsmittel und Reaktionsbedingungen sind z.B. beschrieben in R.C. Larock, Comprehensive Organic Transformation (1989) S. 437-438, VCH Publishers Inc. und der dort angegebenen Literatur. Bevorzugte Reduktionsmittel sind z.B. Bor-oder Aluminiumwasserstoffverbindungen wie BH₃/THF, BH₃/DMS und deren Salze wie NaBH₄. Als Lösungsmittel eignen sich z.B. Ether wie Dioxan oder Tetrahydrofuran. Die Reaktionstemperatur liegt im allgemeinen zwischen 0°C und dem Siedepunkt des Lösungsmittels. Wenn das Reduktionsprodukt anschließend in saurem Medium z.B. Methanol/HCl aufgearbeitet wird, kann die Verbindung der Formel (III) (Verfahrensvariante 2aβ) bzw. die Verbindung der Formel (V) (Verfahrensvarianten 3aα und 4aα) in Form eines Salzes der Formel (IIIa) bzw. (Va) erhalten werden, welches analog zu Verbindung (III) bzw. Verbindung (V) weiter umgesetzt werden kann.

Die Acylierung der Verbindungen der Formel (III) oder (IIIa) nach Verfahrensvariante 1b), 2b) oder 3b) oder der Verbindungen der Formel (V) oder (Va) nach Verfahrensvariante 4aβ) mit einem Sulfonsäurederivat kann unter üblichen Bedingungen für Acylierungsreaktionen zu den Verbindungen der Formel (VI) im Falle von Verbindungen der Formel (V) oder (Va) oder zu den erfindungsgemäßen Verbindungen der Formel (I) im Falle von Verbindungen der Formel (III) oder (IIIa) durchgeführt werden.

Beispielsweise werden Verbindungen der Formel (III) oder (IIIa) bzw. (V) oder (Va) in geeigneten Lösungsmitteln mit Sulfonsäurederivaten in Gegenwart von Basen als Säureakzeptoren zu Verbindungen der Formel (I) bzw. (VI) umgesetzt. Für die Acylierungen geeignete Lösungsmittel sind z.B. Lösungsmittel aus den Gruppen Wasser, Alkohole wie Methanol oder Ethanol, halogenierte aliphatische Kohlenwasserstoffe wie CH₂Cl₂, aromatische Kohlenwasserstoffe wie Toluol, Chlorbenzol oder Xylol, Ether wie Diethylether, Tetrahydrofuran oder Dioxan, Ketone wie Aceton oder Methylisobutylketon, Ester wie Essigsäureethylester, und aprotische Lösungsmittel wie Acetonitril, Dimethylformamid oder Dimethylacetamid, wobei die Lösungsmittel in reiner Form oder als Gemische eingesetzt werden können. Bevorzugt sind Wasser und Gemische aus Wasser und in Wasser löslichen organischen Lösungsmitteln aus den vorstehend angegebenen Gruppen.

Als Basen eignen sich anorganische oder organische Basen, z.B. Carbonate wie K₂CO₃, Na₂CO₃ oder NaHCO₃, Alkali- und Erdalkalihydroxide wie NaOH, KOH oder Ca(OH)₂, oder Amine wie Triethylamin. Die Basen werden im allgemeinen in Mengen von 1-10 Moläquivalenten, vorzugsweise von 1-5 Moläquivalenten pro Verbindung der Formel (III) oder (V) eingesetzt; bei Einsatz von Verbindungen der Formel (IIIa) oder (Va) beträgt die eingesetzte Mindestmenge an Base mindestens zwei Moläquivalente.

Als Sulfonsäurederivate eignen sich z.B. Sulfonsäurehalogenide wie Fluoride, Chloride, Bromide oder Jodide und Sulfonsäureanhydride. Bevorzugt sind Sulfonsäurederivate der Formel R⁷-SO₂-Z, worin R⁷ wie in Formel (I) definiert ist, und Z eine Abgangsgruppe wie Halogen (z.B. Fluor, Chlor, Brom oder Jod) oder O-SO₂-R^{Z} ist, worin R^{Z} wie R⁷ in Formel (I) definiert ist. Die Acylierung erfolgt z.B. derart, daß die Verbindungen der Formel (III) oder (IIIa) bzw. (V) oder (Va) in geeigneten Lösungsmitteln in Gegenwart einer geeigneten Base mit den Sulfonsäurederivaten im allgemeinen bei Temperaturen von -20 bis 100°C umgesetzt werden. Bevorzugt sind Temperaturen von -10 bis 50°C. Die Mengen an Sulfonsäurederivaten liegen im allgemeinen bei 1-10-Moläquivalent, vorzugsweise bei 1-5-Moläquivalent pro Verbindung der Formel (III) oder (IIIa) bzw. (V) oder (Va).

Die vorliegende Erfindung betrifft neben Verbindungen der Formel (I) und deren Herstellung auch deren weitere Umsetzung zu Verbindungen der Formel (VII) und (VIII). Verbindungen der Förmel (I) mit R² und/oder R³ = Acyl sind dabei zunächst nach bekannten Methoden in Verbindungen der Formel (I) mit R² = R³ = H zu überführen, die dann weiter zu Verbindungen der Formel (VII) und (VIII) umgesetzt werden. Die in Formel (VII) und (VIII) verwendeten Symbole haben die gleiche Bedeutung wie in Formel (I) angegeben, einschließlich der hierfür angegebenen Vorzugsbereiche, und Y in Formel (VII) bedeutet Halogen wie Fluor, Chlor, Brom oder Jod. Bevorzugt sind Verbindungen der Formeln (VII) und (VIII), worin die Gruppe -CH₂-NR⁶-SO₂-R⁷ in para-Stellung zur Gruppe -CO-OR¹ steht.

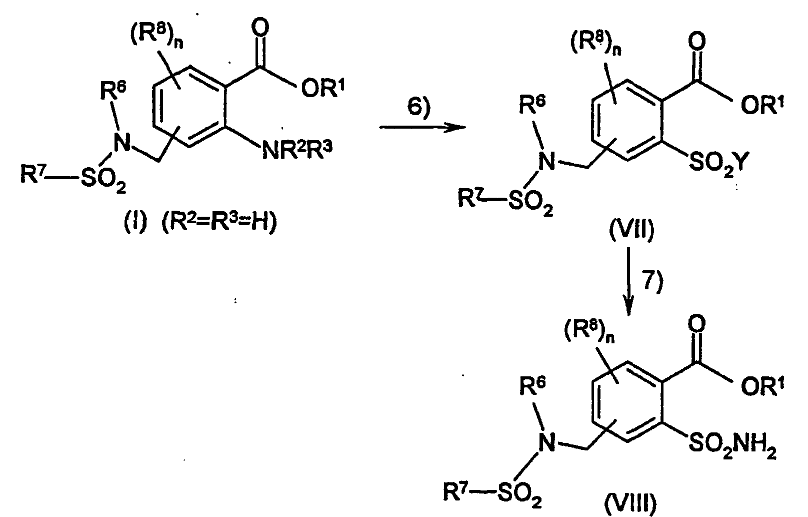

Verbindungen der Formel (VII) und (VIII) sind wie in (EP-A-723 534) beschrieben geeignete Vorstufen zur Herstellung von potenten herbiziden Sulfonylhamstoffen, wobei die Herstellung der Verbindungen der Formeln (VII) und (VIII) im vorliegenden erfindungsgemäßen Verfahren besonders effizient ist und die Verbindungen der Formeln (VII) und (VIII) in sehr guten Ausbeuten und Reinheiten erhalten werden.

Methoden zur Umwandlung 6) von Anilinen zu Sulfonsäurehalogeniden sind bekannt (siehe z.B. H. Meerwein et al., Chem. Berichte 90, 841-852 (1957)). Überraschenderweise gelingt die Umsetzung von Verbindungen der Formel (I) mit R², R³=H zu Verbindungen der Formel (VII) auf Basis von in der Literatur beschriebenen Verfahrensweisen. So lassen sich Verbindungen der Formel (I) mit R², R³=H unter geeigneten Bedingungen diazotieren und anschließend mit geeigneten SO₂-Quellen wie SO₂-Gas, Na₂S₂O₅ oder NaHSO₃ in Gegenwart von Säuren wie Carbonsäuren, z.B. Essigsäure oder Mineralsäuren, z.B. Halogenwasserstoffsäuren HY wie HCl oder HBr und Katalysatoren, z.B. Kupferkatalysatoren auf Basis von Cu^{I} und/oder Cu^{II}-Salzen zu Sulfonsäurehalogeniden der Formel (VII) kuppeln.

Die Diazotierung kann mit geeigneten Diazotierungsmitteln wie NaNO₂ in Gegenwart von Säuren wie Mineralsäuren, vorzugsweise Halogenwasserstoffsäuren HY wie HCl oder HBr durchgeführt werden. Als Lösungsmittel wird vorzugsweise ein Wasser/Säure-Gemisch verwendet, insbesondere ein Gemisch von Wasser/Carbonsäure (z.B. Essigsäure) oder Wasser/ Mineralsäure (z.B. Halogenwasserstoffsäure HY wie HCl oder HBr). Die Reaktionstemperatur beträgt im allgemeinen -20 bis 50°C, vorzugsweise -10 bis 20°C.

Für die anschließende Kupplungsreaktion können als Lösungsmittel z.B. verwendet werden: Wasser, Carbonsäuren wie Essigsäure, Carbonsäureester wie Essigsäureethylester, Ether wie Diethylether, Tetrahydrofuron oder Dioxan, halogenierte aliphatische Kohlenwasserstoffe wie CH₂Cl₂ oder Dichlorethan, aromatische Kohlenwasserstoffe wie Toluol, Chlorbenzol oder Xylol, oder Ketone wie Aceton oder Methylisobutylketon. Außerdem enthält das Reaktionsgemisch Säuren z.B. Carbonsäuren wie Essigsäure oder Mineralsäuren wie Halogenwasserstoffsäuren HY, z.B. HCl oder HBr, die entweder noch aus der Diazotierung stammen und/oder in der Kupplungsreaktion zugegeben werden. Als SO₂-Quelle kann z.B. SO₂-Gas (1-10 Äquivalente), Na₂S₂O₅(1-10 Äquivalente) oder NaHSO₃ (1-10 Äquivalente) verwendet werden, in Gegenwart von Katalysatoren, z.B. Kupferkatalysatoren wie CuCl (1-20mol-%), CuCl₂ (1-20mol-%), CuBr (1-20mol-%) oder CuBr₂ (1-20mol-%).

Ausgehend von Sulfonsäurehalogeniden der Formel (VII) gelingt die Aminolyse 7) zu Sulfonsäureamiden der Formel (VIII) überraschenderweise mit hoher Effizienz und Ausbeute, indem man Verbindungen der Formel (VII) beispielsweise in geeigneten Lösungsmitteln mit Ammoniak umsetzt.

Die Aminolyse kann mit geeigneten Reagentien, z.B. 2-10 Moläquivalent wäßriger Ammoniaklösung oder NH₃-Gas in Gegenwart eines Lösungsmittels z.B. Ketone wie Aceton oder Methylisobutylketon, halogenierte aliphatische Kohlenwasserstoffe wie CH₂Cl₂, aromatische Kohlenwasserstoffe wie Xylol. Toluol oder Chlorbenzol, Ether wie Diethylether, Tetrahydrofuran oder Dioxan, Ester wie Essigsäureethylester, aprotische Lösungsmittel wie Dimethylformamid, Dimethylacetamid oder Acetonitril, oder Gemischen dieser Lösungsmittel durchgeführt werden. Die Reaktionstemperatur beträgt im allgemeinen -10 bis 100°C, vorzugsweise -10 bis 40°C, besonders bevorzugt -10 bis 20°C.

Die Verbindungen der Formel (VII) und (VIII) können anschließend auf verschiedene Weise zu Sulfonylhamstoffen, vorzugsweise zu Sulfonylhamstoffen der Formel (XIII) und/oder deren Salzen umgesetzt werden, z.B. indem man
8) ein Sulfonylhalogenid der Formel (VII) mit einem Cyanat MOCN, worin M ein Ammoniumion oder ein Alkalimetallion wie U, Na oder K ist, und mit einem Aminoheterocyclus der Formel (XII) in Gegenwart einer Base zum Sulfonylhamstoff umsetzt; oder 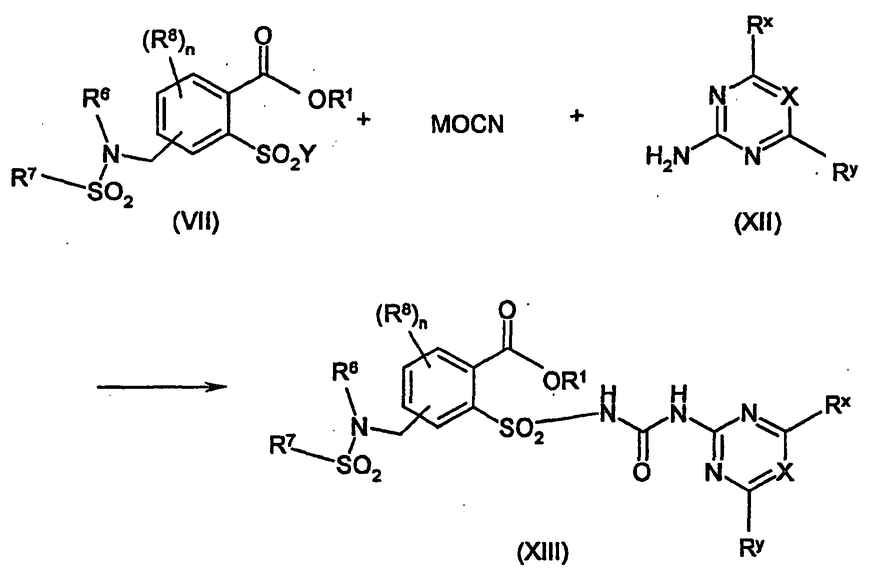
9) eine Verbindung der Formel (VIII) mit einem heterocyclischen Carbamat der Formel (IX), worin Ph unsubstituiertes oder substituiertes Phenyl ist, zum Sulfonylhamstoff umsetzt; oder 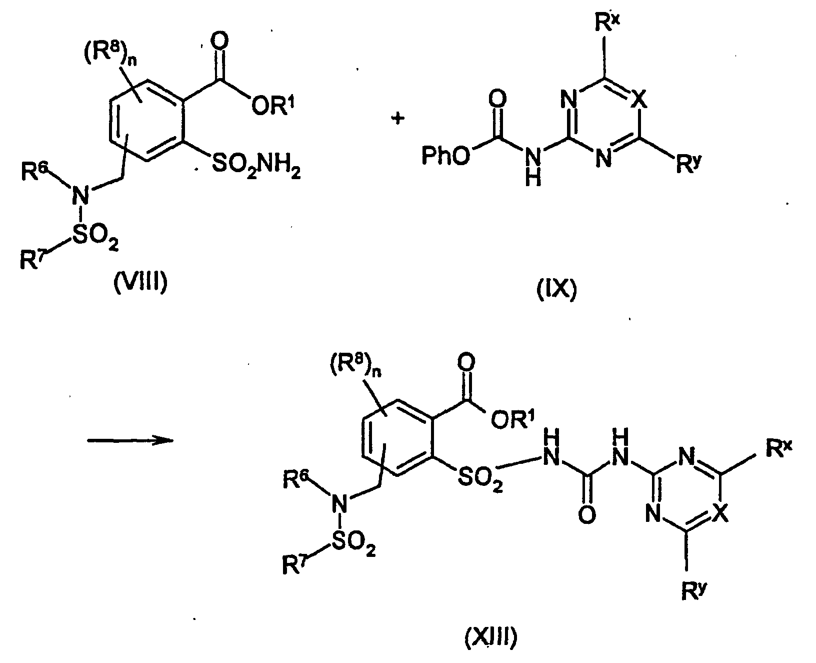
10) a) zunächst einen Aminoheterocyclus der Formel (XII) in Gegenwart einer Base wie Trialkylamin, z.B. Triethylamin, mit Phosgen zu einem Heterocyclylisocyanat der Formel (X) umsetzt, und b) das gebildete Heterocyclylisocyanat der Formel (X) mit einem Phenylsulfonamid der Formel (VIII) zum Sulfonylhamstoff umsetzt; oder 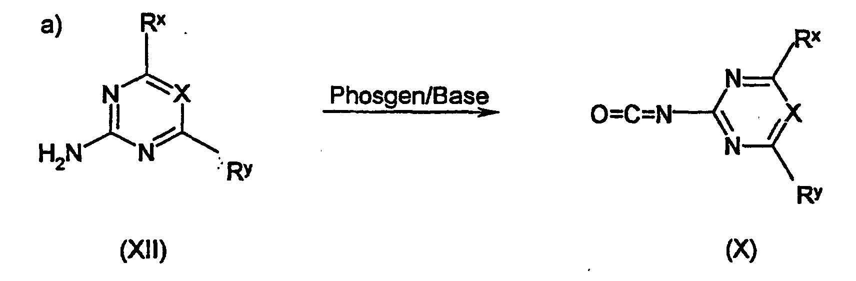 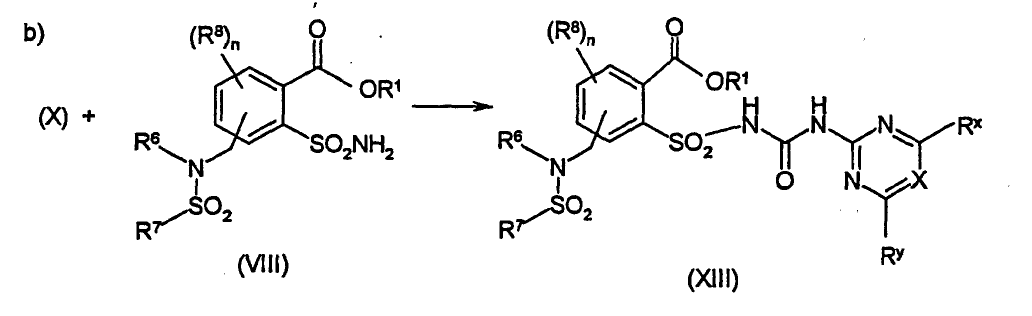
11) a) eine Verbindung der Formel (VIII) mit einem Alkylisocyanat, z.B. RNCO, worin R = C₁-C₁₀-Alkyl ist, und Phosgen zu einem Sulfonylisocyanat der Formel (XI) umsetzt, und b) das gebildete Sulfonylisocyanat der Formel (XI) mit einem Aminoheterocyclus der Formel (XII) zum Sulfonylhamstoff umsetzt; oder 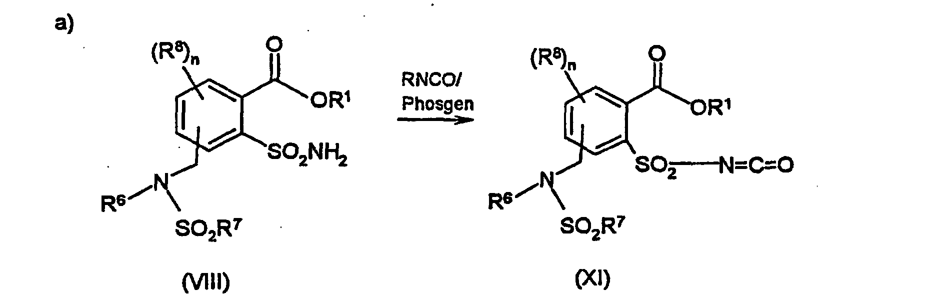 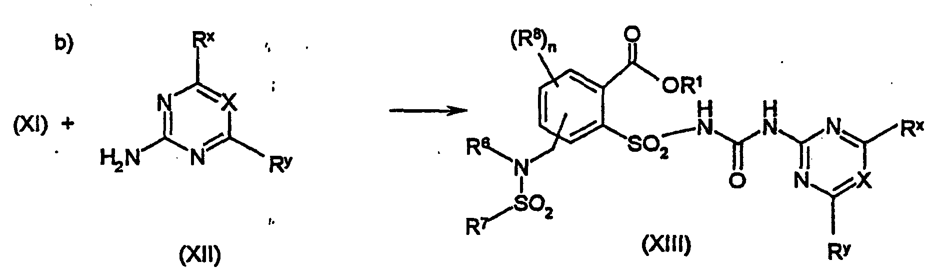
12) a) eine Verbindung der Formel (VIII) mit einem Kohlensäurederivat wie R-CO-OPh, worin Ph = unsubstituiertes oder substituiertes Phenyl ist und R = Halogen oder unsubstituiertes oder substituiertes Phenoxy ist, zu einem Phenylsulfonylcarbamat der Formel (XIV) umsetzt, und b) das gebildete Phenylsulfonylcarbamat der Formel (XIV), worin Ph = unsubstituiertes oder substituiertes Phenyl ist, mit einem Aminoheterocydus der Formel (XII) zum Sulfonylharnstoff umsetzt.

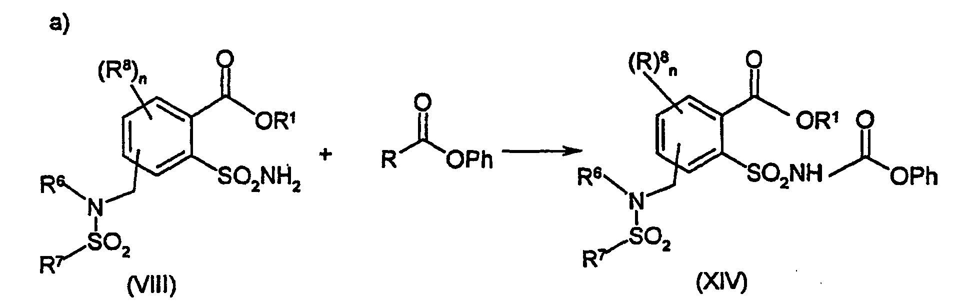

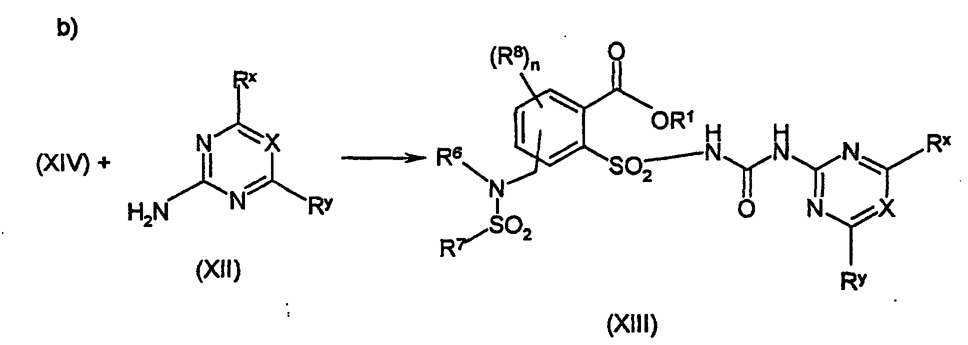

Die in den Formeln (IX), (X), (XI), (XII), (XIII) und (XIV) verwendeten Symbole haben die gleiche Bedeutung wie in Formel (I) angegeben, einschließlich der hierfür angegebenen Vorzugsbereiche, außerdem werden darin noch folgende Bedeutungen verwendet:
- R^{x}, R^{y}: sind unabhängig voneinander ein Wasserstoffatom, Halogen, (C₁-C₄)Alkyl, (C₁-C₄)Alkoxy, (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di[(C₁-C₄)alkyl]amino, (C₂-C₆)Alkenyl, (C₂-C₆)Alkinyl, (C₃-C₆)Alkenyloxy oder (C₃-C₆)Alkinyloxy,
- X: ist CH oder N, und
- Y: ist Halogen wie Fluor, Chlor, Brom oder Jod, vorzugsweise Chlor.

Bevorzugt sind Verbindungen der Formeln (XI), (XIII) und (XIV), worin die Gruppe - CH₂-NR⁶-SO₂-R⁷ in para-Stellung zur Gruppe -COOR¹ steht.

Sulfonylhamstoffe wie die Verbindungen der Formel (XIII) können Salze bilden, bei denen der Wasserstoff der -SO₂-NH-Gruppe durch ein für die Landwirtschaft geeignetes Kation ersetzt ist. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, H₂SO₄ oder HNO₃. Soweit in dieser Beschreibung Sulfonylhamstoffe wie die Verbindungen der Formel (XIII) bezeichnet werden, gelten hierdurch auch jeweils deren Salze als mit umfaßt.

In Verfahrensvariante 8) erfolgt die Umsetzung der Sulfonylhalogenide (VII) mit Aminoheterocyclen der Formel (XII) und Cyanaten MOCN vorzugsweise basenkatalysiert in inerten aprotischen organischen Lösungsmitteln wie Essigsäureethylester, Tetrahydrofuran, Toluol oder Acetonitril zwischen 0°C und dem Siedepunkt des Lösungsmittels. Geeignete Basen sind z.B. organische Aminbasen, insbesondere Pyridine wie Pyridin oder 3-Methylpyridin.

In Verfahrensvariante 9) erfolgt die Umsetzung der Verbindungen der Formeln (VIII) und (IX) vorzugsweise basenkatalysiert in einem inerten organischen Lösungsmittel wie Dichlormethan, Acetonitril, Dioxan, Tetrahydrofuran oder Essigsäurethylester zwischen 0°C und dem Siedepunkt des Lösungsmittels. Als Basen werden beispielsweise K₂CO₃ oder organische Aminbasen wie 1,8-Diazabicyclo[5.4.0]undec-7-en (DBU) verwendet.

In Verfahrensvariante 10) erfolgt die Umsetzung der Verbindung der Formel (XII) mit Phosgen zu Heterocyclylisocyanaten der Formel (X) z.B. in inerten organischen Lösungsmitteln, wie Essigsäureethylester, Dioxan oder aromatischen Lösungsmitteln wie Chlorbenzol, gegebenenfalls unter Zusatz einer organischen Aminbase wie Triethylamin, im allgemeinen zwischen 0°C und dem Siedepunkt des Lösungsmittels. Die anschließende Umsetzung der Verbindung der Formel (X) mit der Verbindung der Formel (VIII) erfolgt z.B. in inerten Lösungsmitteln wie Essigsäureethylester, Dioxan oder aromatischen Lösungsmitteln wie Chlorbenzol, vorzugsweise in Gegenwart von Basen wie K₂CO₃ oder Trialkylaminen wie Triethylamin oder Tributylamin, im allgemeinen bei Temperaturen von -20°C bis zum Siedepunkt des Lösungsmittels (vgl. z.B. EP-A-232 067 oder EP-A-166516).

In Verfahrensvariante 11) erfolgt die Umsetzung der Verbindung der Formel (VIII) mit einem Alkylisocyanat und Phosgen zu Phenylsulfonylisocyanaten der Formel (XI) z.B. in inerten Lösungsmitteln wie Dichlormethan, Acetonitril, Dioxan, Tetrahydrofuran, Toluol oder Chlorbenzol, im allgemeinen bei Temperaturen von 20°C bis zum Siedepunkt des Lösungsmittels. Die anschließende Umsetzung der Verbindung der Formel (XI) mit Aminoheterocyclen der Formel (XII) erfolgt z.B in inerten Lösungsmitteln wie Dichlormethan, Acetonitril, Dioxan, Tetrahydrofuran, Toluol oder Chlorbenzol, im allgemeinen bei Temperaturen von 0°C bis zum Siedepunkt des Lösungsmittels (vgl. z.B. US 4,481,029).

In Verfahrensvariante 12) erfolgt die Umsetzung der Verbindung der Formel (VIII) mit einem Kohlensäurederivat, z.B. Kohlensäurediphenylester oder Chlorameisensäurephenylester, zu einem Phenylsulfonylcarbamat der Formel (XIV) z.B. in inerten Lösungsmitteln wie Xylol, Dichlormethan, Acetonitril, Dioxan, Tetrahydrofuran, Toluol oder Chlorbenzol, vorzugsweise in Gegenwart einer Base wie K₂CO₃ oder organischen Aminbasen wie Triethylamin vorzugsweise bei Temperaturen von 20°C bis zum Siedepunkt des Lösungsmittels (vgl. z.B. US 4,684,393 und US 4,743,290). Die anschließende Umsetzung der Verbindung der Formel (XIV) mit Aminoheterocyclen der Formel (XII) erfolgt z.B. in inerten Lösungsmitteln wie Xylol, Dichlormethan, Acetonitril, Dioxan, Tetrahydrofuran, Toluol oder Chlorbenzol, im allgemeinen bei Temperaturen zwischen 20°C und dem Siedepunkt des Lösungsmittels.

Mit den erfindungsgemäßen Verbindungen der Formel (I) wird somit eine effiziente Herstellung von herbiziden Sulfonylhamstoffen und anderen Wirkstoffen ermöglicht.

### Beispiele

### Beispiel 1

### a) 3-Amino-4-methoxycarbonylbenzylammoniumchlorid

Eine Suspension von 900 g (4,37 mol) 4-Cyano-2-nitrobenzoesäuremethylester in 13,5 l Methanol wird bei Raumtemperatur nach Zugabe von 365 ml konzentrierter Salzsäure (4,37 mol) und 9 g PtO₂ zunächst bei 1 bar Wasserstoffdruck hydriert. Nach abklingender Wasserstoffaufnahme wird der Druck auf 17 bar erhöht und bis zur vollständigen Wasserstoffaufnahme weiterhydriert. Zur Aufarbeitung wird auf Normaldruck entspannt, der Katalysator über Kieselgel abgesaugt und das Filtrat im Vakuum vollständig eingeengt. Digerieren des Rückstandes mit Ethylacetat liefert 3-Amino-4-methoxycarbonylbenzylammoniumchlorid, Ausbeute 757 g (80 %), Schmelzpunkt 185-190°C (Zers.).

### b) 2-Amino-4-methansulfonylaminomethyl-benzoesäuremethylester

3g 3-Amino-4-methoxycarbonylbenzylammoniumchlorid (18,8 mmol) werden in 50 ml Dimethylacetamid gelöst, mit Triethylamin (2,8 g, 27,7 mmol) versetzt und anschließend bei 0-10°C mit einer Lösung von Methansulfonsäurechlorid (1,6 g, 13,8 mmol) in 20 ml Dimethylacetamid umgesetzt. Nach 1 h wird das Lösungsmittel im Vakuum entfernt und der Rückstand mit Wasser/Dichlormethan extraktiv aufgearbeitet. Die vereinigten organischen Extrakte werden nach Waschen mit Wasser und Trocknen (Na₂SO₄) einrotiert. Der erhaltene Rückstand liefert nach Kristallisation aus Wasser 3 g (84 %) 2-Amino-4-methansulfonylaminomethyl-benzoesäuremethylester mit einem Schmelzpunkt 120-121°C.

### c) 2-Chlorsulfonyl-4-methansulfonylaminomethyl-benzoesäuremethylester

Eine Lösung von 3 g (11,6 mmol) 2-Amino-4-methansulfonylaminomethyl-benzoesäuremethylester in 20 ml konzentrierter Salzsäure wird nach Zugabe von 5 ml Eisessig bei 0-5°C in 0,5 h mit einer wäßrigen NaNO₂-Lösung (0,81 g, 11,7 mmol, 10 ml Wasser) versetzt und 0,5 h bei 5°C nachgerührt Parallel hierzu werden 0,34 g (3,5 mmol) CuCl in 30 ml Eisessig, der vorher mit SO₂-Gas gesättigt wurde, suspendiert und anschließend mit 30 ml Toluol versetzt. Zu diesem Gemisch tropft man bei 35°C die zuvor bereitete Diazoniumsalzlösung in 0,5 h unter spontan einsetzender Gasentwicklung hinzu. Nach 1 h wird mit Wasser versetzt, die Phasen getrennt und die wäßrige Phase mit Dichlormethan nachextrahiert. Die vereinigten organischen Phasen werden gewaschen, getrocknet (Na₂SO₄) und im Vakuum eingeengt. Ausrühren des Rückstandes mit Toluol liefert 2,5 g (63 %) 2-Chlorsulfonyl-4-methansulfonylaminomethyl-benzoesäuremethylester mit einem Schmelzpunkt von 93-94°C.

### d) 2-Sulfamoyl-4-methansulfonylaminomethyl-benzosäuremethylester

Eine Lösung von 11 g (32 mmol) 2-Chlorsulfonyl-4-methansulfonylaminomethyl-benzoesäuremethylester in 200 ml THF wird bei 0°C mit 1,1 g (64 mmol) NH₃-Gas versetzt. Zur Aufarbeitung wird im Vakuum eingeengt. Ausrühren des Rückstandes mit Wasser liefert nach Filtration und Trocknung im Vakuum 8,3 g (80 %) 2-Sulfamoyl-4-methansulfonylaminomethyl-benzosäuremethylester mit Schmelzpunkt 185-187°C.

### e) 2-[3-(4,6-Dimethoxypyrimidin-2-yl)ureidosulfonyl]-4-methansulfonaminomethyl-benzoesäuremethylester

87,15 g (0,2677 mol) 2-Sulfamoyl-4-methansutfonylaminomethyl-benzosäuremethylester und 74,42 g (0,2677 mol) N-(4,6-Dimethoxypyrimidin-2-yl)phenylcarbamat werden unter Eiskühlung bei 5°C in 600 ml Acetonitril suspendiert und innerhalb 0,5 h mit 40,4 ml (0,2677 mol) 1,8-Diazabicydo[5.4.0]undec-7en versetzt. Nach 2 h bei Raumtemperatur wird ca. 2/3 des Lösungsmittels im Vakuum entfernt und der Rückstand mit 600 ml 2n HCl und 400 ml Diisopropylether kräftig verrührt. Das ausgefallene Produkt wird abgesaugt, sukzessive mit Wasser und Diisopropylether gewaschen (je 2 mal) und im Vakuum getrocknet. Es werden 125 g 2-[3-(4,6-Dimethoxypyrimidin-2-yl)ureidosulfonyl]-4-methansulfonaminomethylbenzoesäuremethylester (92 %) mit einem Schmelzpunkt von 191-193°C (Zers.) erhalten.

### Beispiel 2

### 3-Amino-4-methoxycarbonylbenzylammoniumchlorid

In einem Hastelloy-Rührautoklaven werden 18,5 g (0,09 mol) 4-Cyano-2-nitrobenzoesäure-methylester und 0,93 g Palladiumhydroxid (20 % auf Kohle) in einer Mischung aus 8 ml konzentrierter Salzsäure (30 %ig) und 315 ml Wasser suspendiert. Dann wird bei einem Wasserstoffdruck von 17 bar bis zur vollständigen Wasserstoffaufnahme hydriert. Nach Entspannen des Autoklaven und Abfiltrieren des Katalysators wird das Filtrat vollständig eingedampft. Man erhält 19,5 g (97 %ig) des 3-Amino-4-methoxycarbonylbenzylammoniumchlorid mit einem Schmelzpunkt von 200-205°C.

### Beispiel 3

### 3-Amino-4-methoxycarbonyl-benzylammoniumchlorid

8,0 g Palladiumhydroxid (20 % auf Kohle) und 100 ml Essigsäure werden in einem Edelstahlautoklaven mit N₂ inertisiert. Dann werden 17 bar Wasserstoff aufgedrückt. Unter gutem Rühren wird nun bei +20°C unter Kühlung eine Lösung von 300 g (1,455 mol) Methyl-2-nitro-4-cyanobenzoat in 2,61 Essigsäure über eine Dosierpumpe während 3 Stunden eindosiert. Der H₂-Druck wird bei 17 bar gehalten. Der Autoklav wird entspannt und mit N₂ inertisiert. Der Katalysator wird abfiltriert und die Filtrate eingedampft. Ausbeute (Acetat des Produktes): 91 % der Theorie in Form eines viskosen Rückstands. Durch Lösen des Rückstandes in Toluol und Einleiten von HCl-Gas (1 Äquivalent) bei 0 bis +10°C erhält man quantitativ eine Fällung von 3-Amino-4-methoxycarbonyl-benzylammoniumchlorid, das in Form weißer Kristalle abfiltriert und getrocknet wird (Ausbeute 91 %, Schmelzpunkt 204-206°C).

### Beispiel 4

### a) 3-Nitro-4-methoxycarbonylbenzylammoniumchlorid

In eine Lösung von 144 g (0,7 mol) 4-Cyano-2-nitrobenzoesäuremethylester in 250 ml THF werden im Laufe einer Stunde bei 40 - 50° C 700 ml einer 1 M BH₃-Lösung in THF (0,7 mol) eindosiert und anschließend noch 1,5 Stunden unter Rückfluß erhitzt. Das Reaktionsgemisch wird dann unter Eiskühlung mit 600 ml mit Chlorwasserstoffgas gesättigtem Methanol versetzt und eine Stunde unter Rückfluß erhitzt. Das Gemisch wird bei Normaldruck vollständig eingeengt und nochmals mit 700 ml Methanol nachgedampft. Der verbliebene Rückstand wird mit 500 ml Ethylacetat verrührt, filtriert und das Produkt getrocknet. Man erhält 115 g (67%) 3-Nitro-4-methoxycarbonylbenzylammoniumchlorid mit einem Schmelzpunkt von 247-248° C.

### b) 4-Methansulfonylaminomethyl-2-nitrobenzoesäuremethylester

Zu einer Lösung von 30,1 g (0,122 mol) 3-Nitro-4-methoxycarbonylbenzylammoniumchlorid und 34 ml (0,244) Triethyamin in 300 ml Dichlormethan dosiert man unter Eiskühlung in 30 min. 9,4 ml (0,244) Methansulfonsäurechlorid und läßt eine Stunde nachrühren. Zur Aufarbeitung wird das Reaktionsgemisch mit Eiswasser versetzt, die Phasen getrennt und die wässrige Phase noch zweimal mit Dichlormethan nachextrahiert. Nach Trocknen (Na₂SO₄), Filtrieren und Einengen verbleiben 31,3 g (89%) 4-Methansulfonylaminomethyl-2-nitrobenzoesäuremethylester als sirupöses Öl.

### c) 2-Amino-4-methansulfonylaminomethylbenzoesäuremethylester

13,25 g (46 mmol) 4-Methansulfonylaminomethyl-2-nitrobenzoesäuremethylester werden in 200 ml Methanol gelöst. Nach Zugabe von 1,4 ml (46 mmol) konz. Salzsäure und 1 g Pd-Katalysator (10 % auf Aktivkohle) wird mit Wasserstoff bei Normaldruck bis zur vollständigen Wasserstoffaufnahme hydriert. Der Ansatz wird vom Katalysator filtriert und das Filtrat vollständig eingeengt. Kristallisation des Rückstandes aus Wasser liefert 10,4 g (88%) 2-Amino-4-methansulfonylaminomethylbenzoesäuremethylester mit einem Schmelzpunkt von 121-122° C.

## Patentansprüche

1. Verbindung der Formel (I), 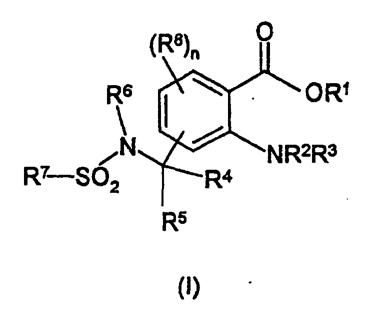 worin
R¹ H, (C₁-C₈)Alkyl, (C₃-C₈)Alkenyl oder (C₃-C₈)Alkinyl ist, wobei die letzten 3 Reste unsubstituiert oder substituiert sind,
R², R³ unabhängig voneinander H oder Acyl sind,
R⁴, R⁵ H sind,
R⁶ H oder (C₁-C₈)Alkyl ist, welches unsubstituiert oder substituiert ist,
R⁷ (C₁-C₈)Alkyl, (C₃-C₈)Alkenyl, (C₃-C₈)Alkinyl, (C₆-C₁₄)-Aryl oder Mono- oder Di-(C₁-C₈)Alkylamino ist, welche unsubstituiert oder substituiert sind, oder
R⁶ und R⁷ gemeinsam eine Kette der Formel -(CH₂)ₘB_{m¹}-bilden, welche unsubstituiert oder substituiert ist, und worin m=2, 3 oder 4, m¹=0 oder 1 und B=CO oder SO₂ bedeutet,
R⁸ gleich oder verschieden (C₁-C₄)Alkyl, (C₁-C₄)Alkoxy, [(C₁-C₄)Alkyl]carbonyl oder [(C₁-C₄)Alkoxy]carbonyl sind, welche unsubstituiert oder substituiert sind, oder R⁸ ist Halogen, NO₂ oder CN, und
n ist gleich 0, 1, 2 oder 3.

2. Verbindung der Formel (I) gemäß Anspruch 1, worin
R¹ H oder (C₁-C₄)-Alkyl, vorzugsweise (C₁-C₄)Alkyl ist,
R², R³ H sind,
R⁴, R⁵ H sind,
R⁶ H ist,
R⁷ (C₁-C₄)Alkyl ist, und
n gleich 0 ist.

3. Verbindung der Formel (I) gemäß Anspruch 1 oder 2, worin die Gruppe -CR⁴R⁵-NR⁶-SO₂-R⁷ in para-Stellung zur Gruppe -CO-OR¹ steht.

4. Verfahren zur Herstellung einer Verbindung der Formel (I) gemäß einem oder mehreren der Ansprüche 1-3, enthaltend die Schritte, worin
1a) eine Verbindung der Formel (II) 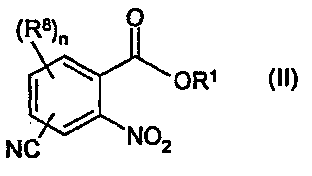 durch katalytische Hydrierung in Abwesenheit einer Säure zu einer Verbindung der Formel (III) oder durch katalytische Hydrierung in Gegenwart einer Säure H⁺X⁻, wobei X⁻ ein Äquivalent eines Säureanions ist, zu einer Verbindung der Formel (IIIa), worin X⁻ ein Äquivalent eines Säureanions ist, umgesetzt wird, 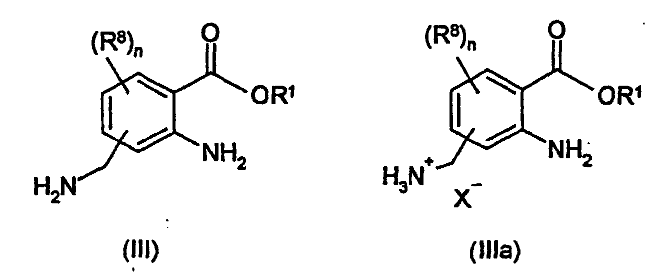 und anschließend
1b) die Verbindung der Formel (III) oder (IIIa) mit einem Sulfonsäurederivat zu einer Verbindung der Formel (I) mit R², R³ und R⁶=H umgesetzt wird; oder
2a)
α) eine Verbindung der Formel (II) 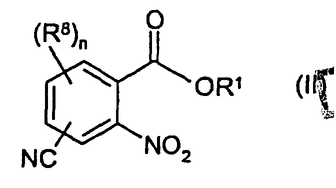 durch gängige Reduktionsverfahren für Nitroverbindungen zu einer Verbindung der Formel (IV) umgesetzt wird, 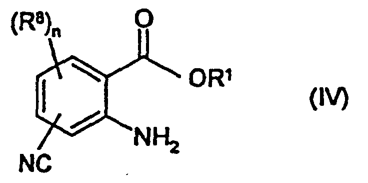 und anschließend
β) die Verbindung der Formel (IV) entweder durch katalytische Hydrierung oder durch gängige Reduktionsverfahren für Nitrile, zu einer Verbindung der Formel (III) oder (IIIa) umgesetzt wird,
und anschließend
2b) die Verbindung der Formel (III) oder (IIIa) mit einem Sulfonsäurederivat zu einer Verbindung der Formel (I) mit R², R³ und R⁶=H umgesetzt wird; oder
3a)
α) eine Verbindung der Formel (II) 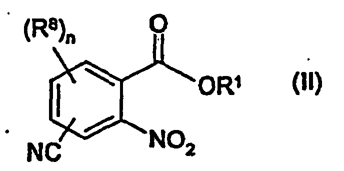 durch gängige Reduktionsverfahren für Nitrile zu einer Verbindung der Formel (V) oder (Va), worin X⁻ wie in Formel (IIIa) definiert ist, umgesetzt wird, . 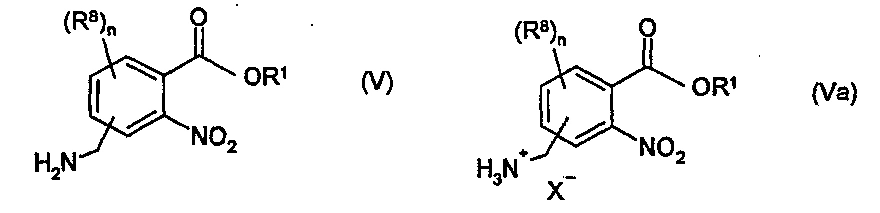 und anschließend
β) die Verbindung der Formel (V) oder (Va) durch gängige Reduktionsverfahren für Nitroverbindungen oder durch katalytische Hydrierung zu einer Verbindung der Formel (III) oder (IIIa) umgesetzt wird,
und anschließend
3b) die Verbindung der Formel (III) oder (IIIa) mit einem Sulfonsäurederivat zu einer Verbindung der Formel (I) mit R², R³ und R⁶=H umgesetzt wird; oder
4a)
α) eine Verbindung der Formel (II) 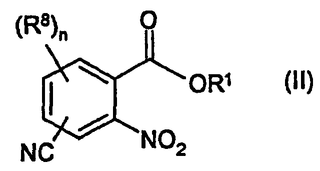 durch gängige Reduktionsverfahren für Nitrile zu einer Verbindung der Formel (V) oder (Va), worin X⁻ wie in Formel (IIIa) definiert ist, umgesetzt wird, 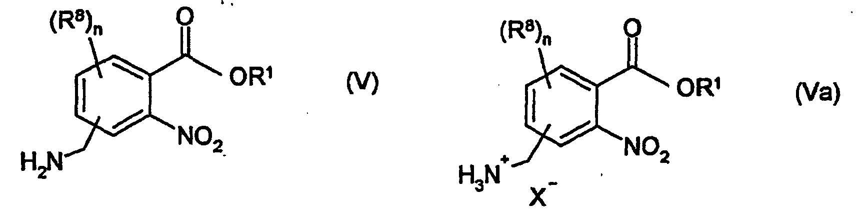
β) und anschließend die Verbindung der Formel (V) oder (Va) mit einem Sulfonsäurederivat zu einer Verbindung der Formel (VI) umgesetzt wird, 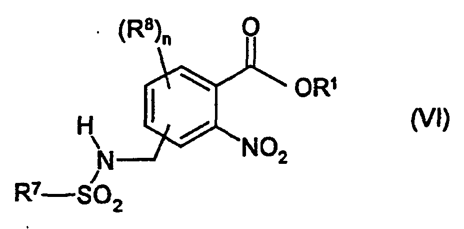
und anschließend
4b) die Verbindung der Formel (VI) durch gängige Reduktionsverfahren für Nitroverbindungen oder durch katalytische Hydrierung zu einer Verbindung der Formel (I) mit R², R³ und R⁶=H umgesetzt wird,
wobei R¹, R⁷, R8 und n in den Formeln (II), (III), (IIIa), (IV), (V), (Va) und (VI) wie in Formel (I) in Anspruch 1 oder 2 definiert sind; und optional anschließend
5) die in einem der Schritte 1)-4) erhaltene Verbindung der Formel (I) mit einem Alkylierungsmittel oder durch reduktive Aminierung zu einer Verbindung der Formel (I) mit R⁶=unsubstituiertes oder substituiertes C₁-C₈-Alkyl, oder mit einem Acylierungsmittel zu einer Verbindung der Formel (I) mit R² und/oder R³=Acyl umgesetzt wird.

5. Verfahren zur Herstellung einer Verbindung der Formel (XIII), worin R¹, R⁶, R⁷, R⁸ und n wie in Formel (I) in Anspruch 1 oder 2 definiert sind, R^{x}, R^{y} unabhängig voneinander ein Wasserstoffatom, Halogen, (C₁-C₄)Alkyl, (C₁-C₄)Alkoxy, (C₁-C₄)Alkylthio sind, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di[(C₁-C₄)alkyl]amino, (C₂-C₆)Alkenyl, (C₂-C₆)Alkinyl, (C₃-C₆)Alkenyloxy oder (C₃-C₆)Alkinyloxy sind, und
X CH oder N ist, 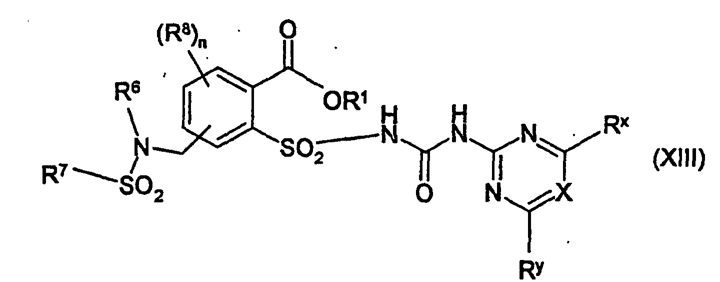
enthaltend die Schritte, worin
**A)** eine Verbindung der Formel (I) wie in Anspruch 1 oder 2 definiert und wobei R² und R³ = H sind, hergestellt wird, indem
1a) eine Verbindung der Formel (II) 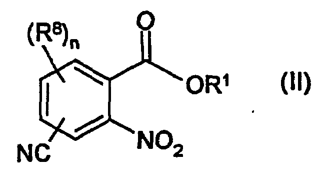 durch katalytische Hydrierung in Abwesenheit einer Säure zu einer Verbindung der Formel (III) oder durch katalytische Hydrierung in Gegenwart einer Säure H⁺X⁻, wobei X⁻ ein Äquivalent eines Säureanions ist, zu einer Verbindung der Formel (IIIa), worin X⁻ ein Äquivalent eines Säureanions ist, umgesetzt wird, 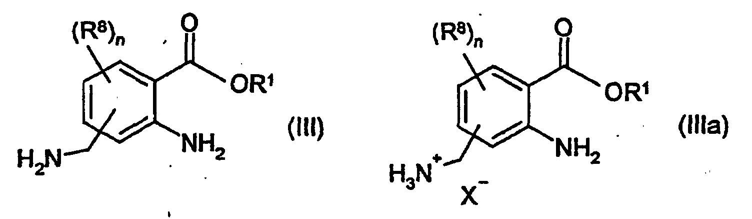 und anschließend
1b) die Verbindung der Formel (III) oder (IIIa) mit einem Sulfonsäurederivat zu einer Verbindung der Formel (I) mit R², R³ und R⁶=H umgesetzt wird; oder
2a)
α) eine Verbindung der Formel (II) 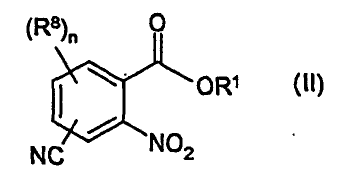 durch gängige Reduktionsverfahren für Nitroverbindungen zu einer Verbindung der Formel (IV) umgesetzt wird, 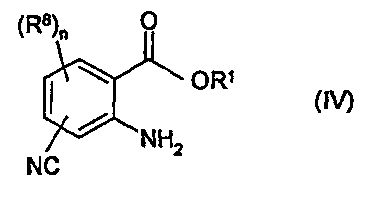 und anschließend
β) die Verbindung der Formel (IV) entweder durch katalytische Hydrierung oder durch gängige Reduktionsverfahren für Nitrile, zu einer Verbindung der Formel (III) oder (IIIa) umgesetzt wird,
und anschließend
2b) die Verbindung der Formel (III) oder (IIIa) mit einem Sulfonsäurederivat zu einer Verbindung der Formel (I) mit R², R³ und R⁶=H umgesetzt wird; oder
3a)
α) eine Verbindung der Formel (II) 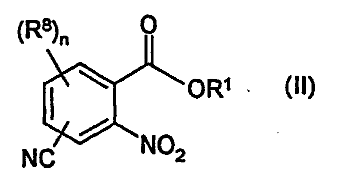 durch gängige Reduktionsverfahren für Nitrile zu einer Verbindung der Formel (V) oder (Va), worin X⁻ wie in Formel (IIIa) definiert ist, umgesetzt wird, 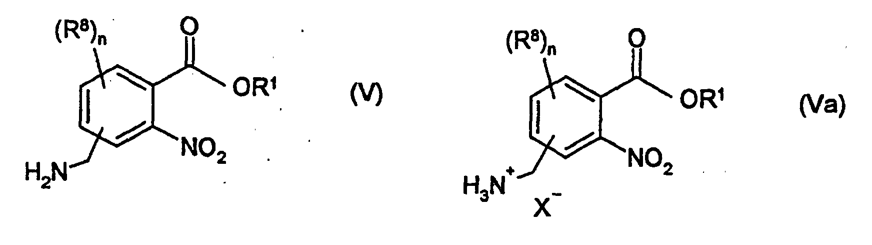 und anschließend
β) die Verbindung der Formel (V) oder (Va) durch gängige Reduktionsverfahren für Nitroverbindungen oder durch katalytische Hydrierung zu einer Verbindung der Formel (III) oder (IIIa) umgesetzt wird,
und anschließend
3b) die Verbindung der Formel (III) oder (IIIa) mit einem Sulfonsäurederivat zu einer Verbindung der Formel (I) mit R², R³ und R⁶=H umgesetzt wird; oder
4a)
α) eine Verbindung der Formel (II) 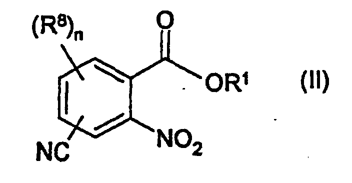 durch gängige Reduktionsverfahren für Nitrile zu einer Verbindung der Formel (V) oder (Va), worin X⁻ wie in Formel (IIIa) definiert ist, umgesetzt wird, 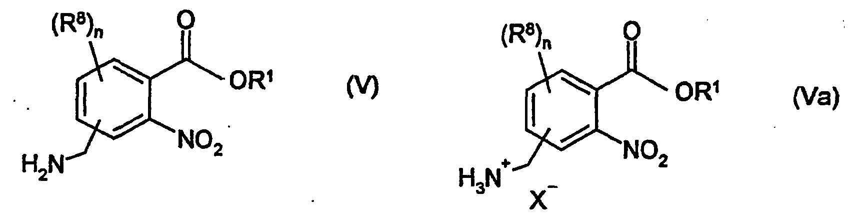
β) und anschließend die Verbindung der Formel (V) oder (Va) mit einem Sulfonsäurederivat zu einer Verbindung der Formel (VI) umgesetzt wird, 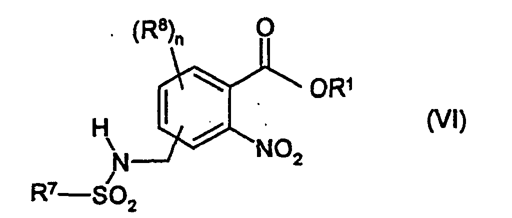 und anschließend
4b) die Verbindung der Formel (VI) durch gängige Reduktionsverfahren für Nitroverbindungen oder durch katalytische Hydrierung zu einer Verbindung der Formel (I) mit R², R³ und R⁶=H umgesetzt wird;
wobei R¹, R⁷, R⁸ und n in den Formeln (II), (III), (IIIa), (IV), (V), (Va), und (VI) wie in Formel (I) in Anspruch 1 oder 2 definiert sind; und optional anschließend
5) die in einem der Schritte A1)-A4) erhaltene Verbindung der Formel (I) mit einem Alkylierungsmittel oder durch reduktive Aminierung zu einer Verbindung der Formel (I) mit R⁶ = unsubstituiertes oder substituiertes C₁-C₈-Alkyl umgesetzt wird; und anschließend
**B)** eine Verbindung der Formel (VII) hergestellt wird, indem
6) die in Schritt A) erhaltene Verbindung der Formel (I) zu einer Verbindung der Formel (VII) umgesetzt wird, 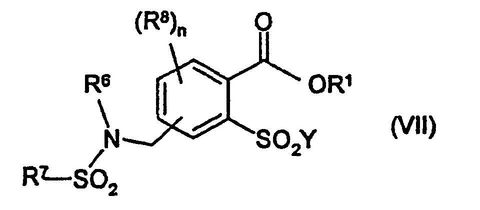 wobei die Verbindung der Formel (I) in Gegenwart einer Säure mit einem Diazotierungsmittel und anschließend mit einer SO₂-Quelle in Gegenwart eines Kupferkatalysators und einer Säure umgesetzt wird, wobei R¹, R⁶, R⁷, R⁸ und n in Formel (VII) wie in Formel (I) in Anspruch 1 oder 2 definiert sind und Y = Halogen ist; und anschließend
**C)** die Verbindung der Formel (XIII) hergestellt wird, indem
**C1)** die in Schritt B6) erhaltene Verbindung der Formel (VII) in Gegenwart von MOCN wobei M ein Ammoniumion oder ein Alkalimetallion ist, mit einem Amin der Formel (XII) zu einer Verbindung der Formel (XIII) umgesetzt wird; oder 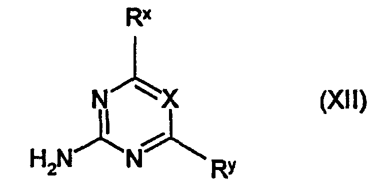
**C 2.1)** eine Verbindung der Formel (VIII) hergestellt wird, indem
7) die in Schritt B6) erhaltene Verbindung der Formel (VII) zu einer Verbindung der Formel (VIII) umgesetzt wird, 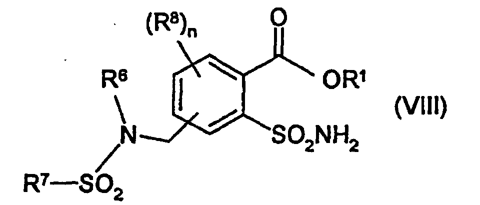 wobei die Verbindung der Formel (VIII) in einem geeigneten Lösungsmittel mit Ammoniak aminolysiert wird, wobei R¹, R⁶, R⁷, R⁸ und n in Formel (VIII) wie in Formel (I) in Anspruch 1 oder 2 definiert sind;
und anschließend
**C2.2)** die Verbindung der Formel (XIII) hergestellt wird, indem
9) die Verbindung der Formel (VIII) mit einer Verbindung der Formel (IX) zu der Verbindung der Formel (XIII) umgesetzt wird; oder 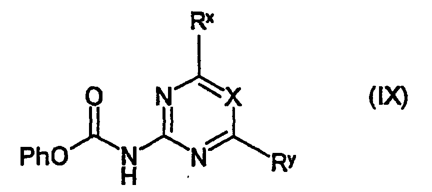
10) die Verbindung der Formel (VIII) mit einem Isocyanat der Formel (X) zu der Verbindung der Formel (XIII) umgesetzt wird; oder 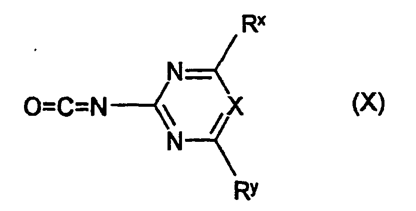
11) die Verbindung der Formel (VIII) mit einem Alkylisocyanat und Phosgen zu einer Verbindung der Formel (XI) umgesetzt wird, 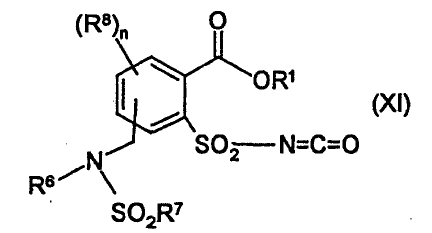 welche anschließend mit einem Amin der Formel (XII) zu der Verbindung der Formel (XIII) umgesetzt wird; oder 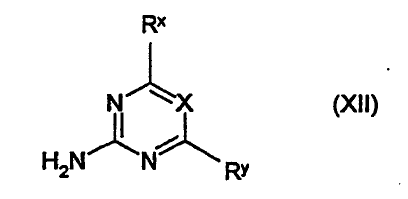
12) die Verbindung der Formel (VIII) mit einem Kohlensäurederivat R-CO-OPh, worin Ph = unsubstituiertes oder substituiertes Phenyl ist und R = Halogen oder unsubstituiertes oder substituiertes Phenoxy ist, zu einer Verbindung der Formel (XIV) umgesetzt wird, 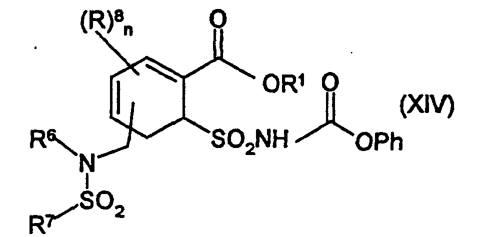 welche anschließend mit einem Amin der Formel (XII) zu der Verbindung der Formel (XIII) umgesetzt wird; 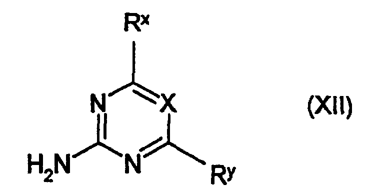
wobei R¹, R⁶, R⁷, R⁸ und n in den Formeln (XI) und (XIV) wie in Formel (I) in Anspruch 1 oder 2 definiert sind, R^{x}, R^{y}, und X in den Formeln (IX), (X) und (XII) wie in Formel (XIII) dieses Anspruchs definiert sind und Ph in den Formeln (IX) und (XIV) unsubstituiertes oder substituiertes Phenyl bedeuten.

6. Verfahren zur Herstellung einer Verbindung der Formel (XIII) wie in Anspruch 5 definiert, 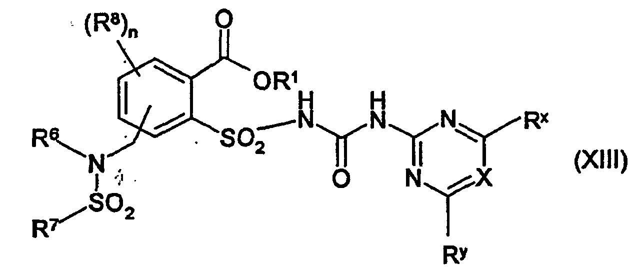 enthaltend die Schritte B) und C1) oder B), C2.1) und C2.2) wie in Anspruch 5 definiert.

7. Verfahren zur Herstellung einer Verbindung der Formel (VII) wie in Anspruch 5 definiert, 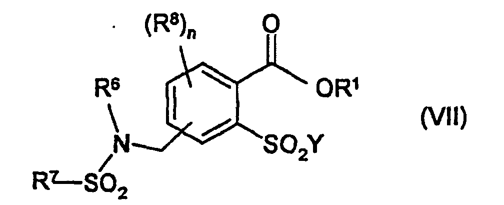 worin
eine Verbindung der Formel (I) wie in Anspruch 1 oder 2 definiert und worin R², R³ = H sind, in Gegenwart einer Säure diazotiert und anschließend mit einer SO₂-Quelle in Gegenwart eines Kupferkatalysators und einer Säure umgesetzt wird.

8. Verfahren zur Herstellung einer Verbindung der Formel (VIII) wie in Anspruch 5 definiert, 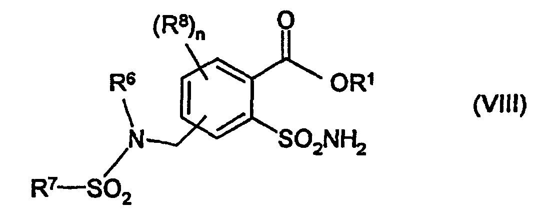 enthaltend die Schritte, worin
A) eine Verbindung der Formel (I) wie in Anspruch 1 definiert und worin R², R³ = H sind, zu einer Verbindung der Formel (VII) umgesetzt wird, 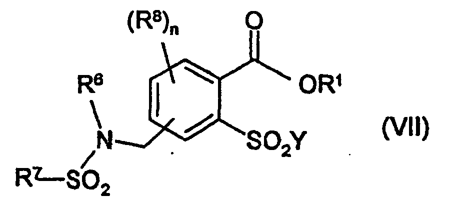 wobei die Verbindung der Formel (I) in Gegenwart einer Säure diazotiert und anschließend mit einer SO₂-Quelle in Gegenwart eines Kupferkatalysators und einer Säure umgesetzt wird,
und anschließend
B) die Verbindung der Formel (VII) zu einer Verbindung der Formel (VIII) umgesetzt wird, 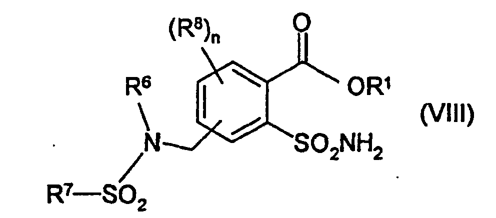 wobei die Verbindung der Formel (VIII) in einem geeigneten Lösungsmittel mit Ammoniak aminolysiert wird.

9. Verwendung einer Verbindung der Formel (II), (III), (IIIa), (IV), (V) oder (Va) wie in Anspruch 4 definiert, zur Herstellung einer Verbindung der Formel (I) wie in Anspruch 1 oder 2 definiert.

10. Verwendung einer Verbindung der Formel (I) wie in Anspruch 1 oder 2 definiert, zur Herstellung eines Sulfonylhamstoffs.

11. Verwendung einer Verbindung der Formel (I) wie in Anspruch 1 oder 2 definiert, zur Herstellung einer Verbindung der Formel (VII), (VIII) oder (XIII) wie in Anspruch 5 definiert.

12. Verbindung der Formel (VI) 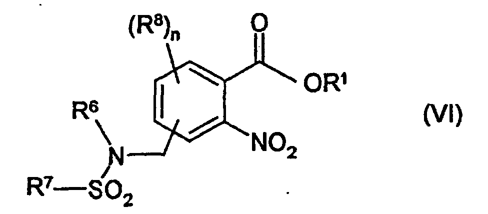 worin R¹, R⁸, R⁷, R⁸ und n wie in Formel (I) gemäß Anspruch 1 oder 2 definiert sind.

13. Verwendung einer Verbindung der Formel (VI) wie in Anspruch 12 definiert, zur Herstellung eines Sulfonylhamstoffs.

14. Verwendung einer Verbindung der Formel (VI) wie in Anspruch 12 definiert, zur Herstellung einer Verbindung der Formel (I) wie in Anspruch 1 oder 2 definiert oder einer Verbindung der Formel (VII), (VIII) oder (XIII) wie in Anspruch 5 definiert.

15. Verbindung der Formel (Z) oder (Za) 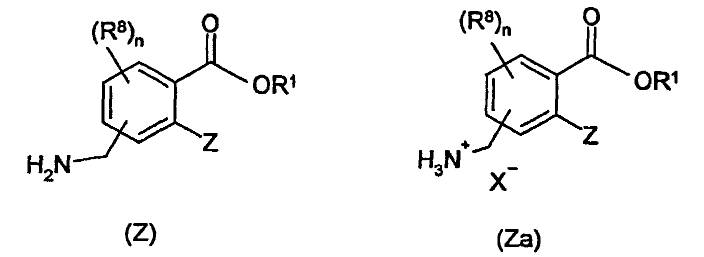 worin R¹, R⁸ und n wie in Formel (I) gemäß Anspruch 1 oder 2 definiert sind und Z gleich NH₂ oder NO₂ ist.

16. Verwendung einer Verbindung der Formel (Z) oder (Za) wie in Anspruch 15 definiert, zur Herstellung eines Sulfonylhamstoffs.

17. Verwendung einer Verbindung der Formel (Z) oder (Za) wie in Anspruch 15 definiert, zur Herstellung einer Verbindung der Formel (I) wie in Anspruch 1 oder 2 definiert oder einer Verbindung der Formel (VII), (VIII) oder (XIII) wie in Anspruch 5 definiert.

## Claims

1. A compound of the formula (I), 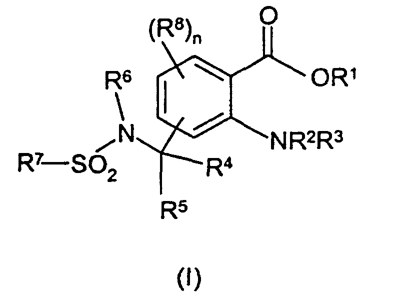 in which
R¹ is H, (C₁-C₈)alkyl, (C₃-C₈)alkenyl or (C₃-C₈)alkynyl, where the last 3 radicals are unsubstituted or substituted,
R², R³ independently of one another are H or acyl,
R⁴, R⁵ are H,
R⁶ is H or (C₁-C₈)alkyl which is unsubstituted or substituted,
R⁷ is (C₁-C₈)alkyl, (C₃-C₈)alkenyl, (C₃-C₈)alkynyl, (C₆-C₁₄)aryl or mono- or di-(C₁-C₈)alkylamino which are unsubstituted or substituted, or
R⁶ and R⁷ together form a chain of the formula -(CH₂)ₘBₘ-
which is unsubstituted or substituted, and where m=2, 3 or 4, m¹=0 or 1 and B=CO or SO₂,
R⁸ radicals are identical or different and are (C₁-C₄)alkyl, (C₁-C₄)alkoxy, [(C₁-C₄)alkyl]carbonyl or [(C₁-C₄)alkoxy]carbonyl which are unsubstituted or substituted, or R⁸ is halogen, NO₂ or CN, and
n is 0, 1, 2 or 3.

2. A compound of the formula (I) as claimed in claim 1, where
R¹ is H or (C₁-C₄)alkyl, preferably(C₁-C₄)alkyl,
R², R³ are H,
R⁴, R⁵ are H,
R⁶ is H,
R⁷ is (C₁-C₄)alkyl, and
n is 0.

3. A compound of the formula (I) as claimed in claim 1 or 2, in which the group -CR⁴R⁵-NR⁶-SO₂-R⁷ is in the para position relative to the group -CO-OR¹.

4. A process for the preparation of a compound of the formula (I) as claimed in one or more of claims 1-3, comprising the steps of
1a) reacting a compound of the formula (II) 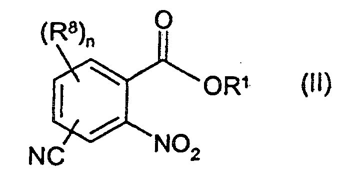 by catalytic hydrogenation in the absence of an acid to give a compound of the formula (III) or by catalytic hydrogenation in the presence of an acid H⁺X⁻, where X⁻ is an equivalent of an acid anion, to give a compound of the formula (IIIa), where X⁻ is an equivalent of an acid anion, 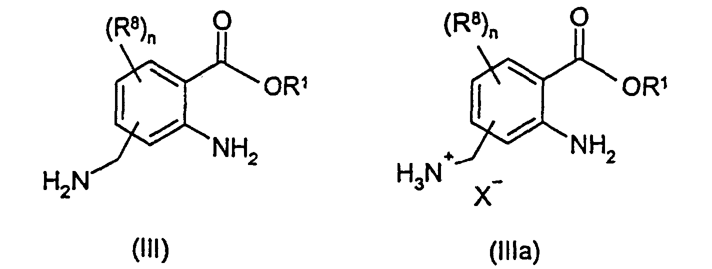 and subsequently
1b) reacting the compound of the formula (III) or (IIIa) with a sulfonic acid derivative to give a compound of the formula (I) where R², R³ and R⁶=H; or
2a)
α) reacting a compound of the formula (II) 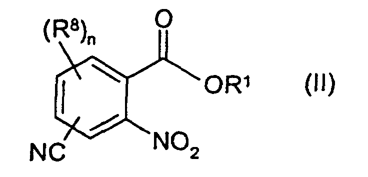 by customary reduction methods for nitro compounds to give a compound of the formula (IV), 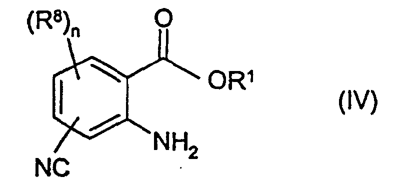 and subsequently
β) reacting the compound of the formula (IV) either by catalytic hydrogenation or by customary reduction methods for nitriles to give a compound of the formula (III) or (IIIa),
and subsequently
2b) reacting the compound of the formula (III) or (IIIa) with a sulfonic acid derivative to give a compound of the formula (I) where R², R³ and R⁶=H; or
3a)
α) reacting a compound of the formula (II) 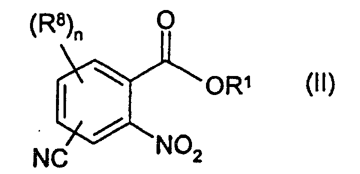 by customary reduction methods for nitriles to give a compound of the formula (V) or (Va), where X⁻ is as defined in formula (IIIa), 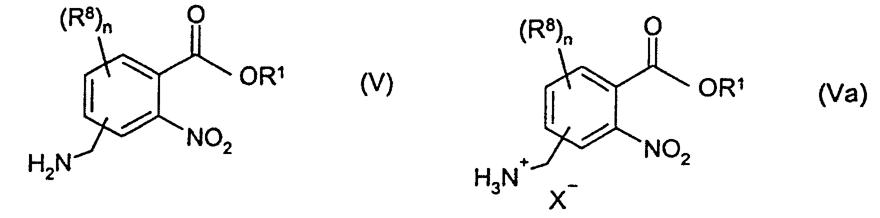 and subsequently
β) reacting the compound of the formula (V) or (Va) by customary reduction methods for nitro compounds or by catalytic hydrogenation to give a compound of the formula (III) or (IIIa),
and subsequently
3b) reacting the compound of the formula (III) or (IIIa) with a sulfonic acid derivative to give a compound of the formula (I) where R², R³ and R⁶=H; or
4a)
α) reacting a compound of the formula (II) 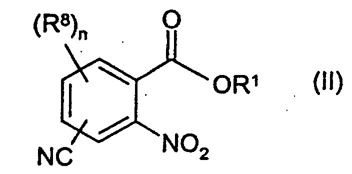 by customary reduction methods for nitriles to give a compound of the formula (V) or (Va), where X⁻ is as defined in formula (IIIa), 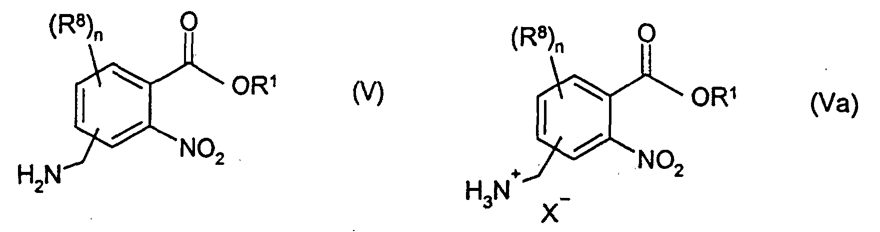
β) and subsequently reacting the compound of the formula (V) or (Va) with a sulfonic acid derivative to give a compound of the formula (VI), 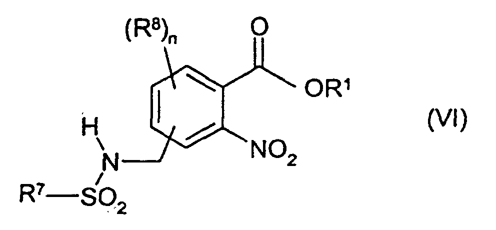
and subsequently
4b) reacting the compound of the formula (VI) by customary reduction methods for nitro compounds or by catalytic hydrogenation to give a compound of the formula (I) where R², R³ and R⁶=H,
where R¹, R⁷, R⁸ and n in formulae (II), (III), (IIIa), (IV), (V), (Va) and (VI) are as defined in formula (I) in claim 1 or 2; and optionally subsequently
5) reacting the compound of the formula (I) obtained in one of steps 1)-4) with an alkylating agent or by reductive amination to give a compound of the formula (I) where R⁶=unsubstituted or substituted C₁-C₈-alkyl, or with an acylating agent to give a compound of the formula (I) where R² and/or R³=acyl.

5. A process for the preparation of a compound of the formula (XIII), in which R¹, R⁶, R⁷, R⁸ and n are as defined in formula (I) in claim 1 or 2,
R^{x}, R^{y} independently of one another are a hydrogen atom, halogen, (C₁-C₄)alkyl, (C₁-C₄)alkoxy, (C₁-C₄)alkylthio, where each of the last-mentioned 3 radicals is unsubstituted or substituted by one or more radicals selected from the group consisting of halogen, (C₁-C₄)alkoxy and (C₁-C₄)alkylthio, or are mono- or di[(C₁-C₄)alkyl]amino, (C₂-C₆)alkenyl, (C₂-C₆)alkynyl, (C₃-C₆)alkenyloxy or (C₃-C₆)alkynyloxy, and
X is CH or N, 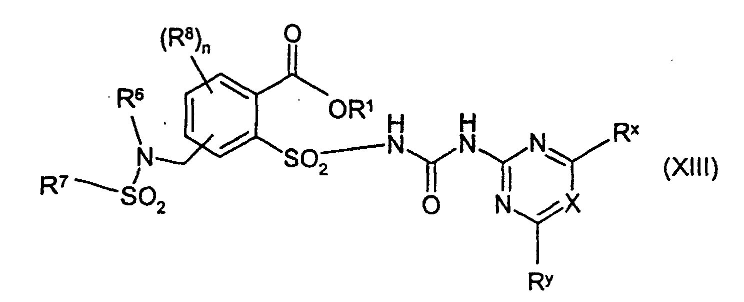
comprising the steps of
**A)** preparing a compound of the formula (I) as defined in claim 1 or 2, where R² and R³ = H, by
1a) reacting a compound of the formula (II) 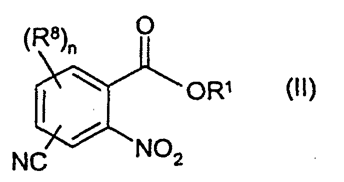 by catalytic hydrogenation in the absence of an acid to give a compound of the formula (III) or by catalytic hydrogenation in the presence of an acid H⁺X⁻, where X⁻ is an equivalent of an acid anion, to give a compound of the formula (IIIa), where X⁻ is an equivalent of an acid anion, 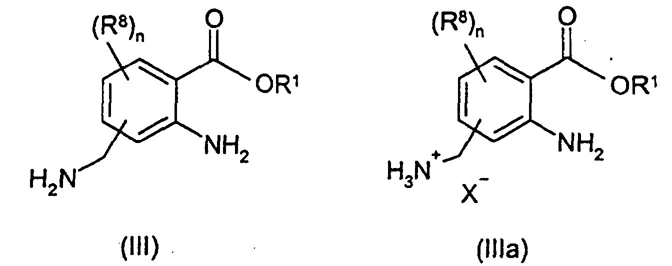 and subsequently
1b) reacting the compound of the formula (III) or (IIIa) with a sulfonic acid derivative to give a compound of the formula (I) where R², R³ and R⁶=H; or
2a)
α) reacting a compound of the formula (II) 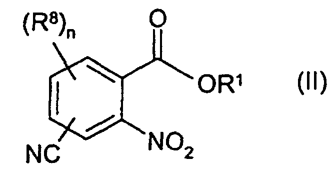 by customary reduction methods for nitro compounds to give a compound of the formula (IV), 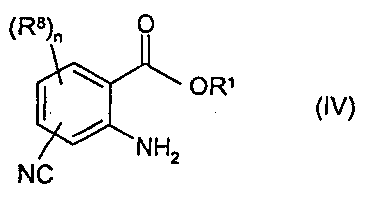 and subsequently
β) reacting the compound of the formula (IV) either by catalytic hydrogenation or by customary reduction methods for nitriles to give a compound of the formula (III) or (IIIa),
and subsequently
2b) reacting the compound of the formula (III) or (IIIa) with a sulfonic acid derivative to give a compound of the formula (I) where R², R³ and R⁶=H; or
3a)
α) reacting a compound of the formula (II) 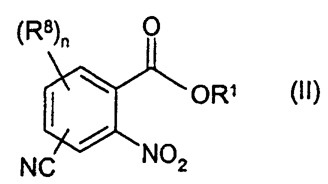 by customary reduction methods for nitriles to give a compound of the formula (V) or (Va), where X⁻ is as defined in formula (IIIa), 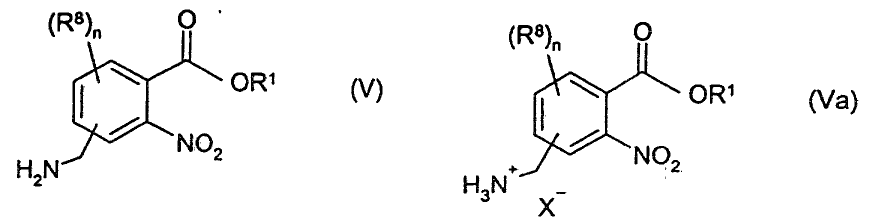 and subsequently
β) reacting the compound of the formula (V) or (Va) by customary reduction methods for nitro compounds or by catalytic hydrogenation to give a compound of the formula (III) or (IIIa),
and subsequently
3b) reacting the compound of the formula (III) or (IIIa) with a sulfonic acid derivative to give a compound of the formula (I) where R², R³ and R⁶=H; or
4a)
α) reacting a compound of the formula (II) 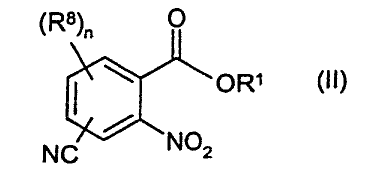 by customary reduction methods for nitriles to give a compound of the formula (V) or (Va), where X⁻ is as defined in formula (IIIa), 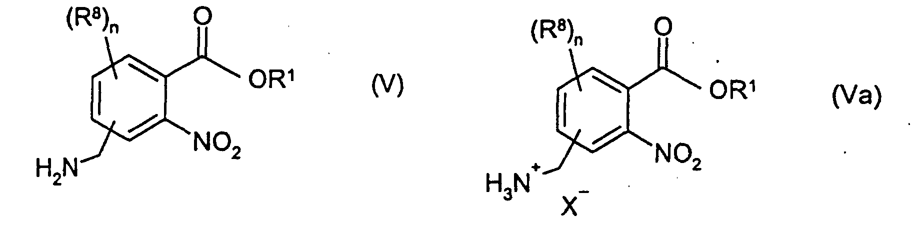
β) and subsequently reacting the compound of the formula (V) or (Va) with a sulfonic acid derivative to give a compound of the formula (VI), 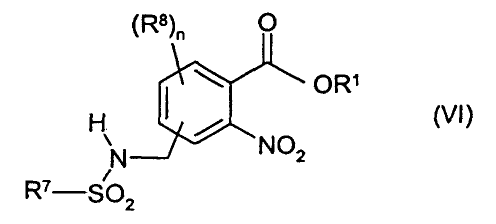
and subsequently
4b) reacting the compound of the formula (VI) by customary reduction methods for nitro compounds or by catalytic hydrogenation to give a compound of the formula (I) where R², R³ and R⁶=H;
where R¹, R⁷, R⁸ and n in formulae (II), (III), (IIIa), (IV), (V), (Va) and (VI) are as defined in formula (I) in claim 1 or 2; and optionally subsequently
5) reacting the compound of the formula (I) which has been obtained in one of steps A1)-A4) with an alkylating agent or by reductive amination to give a compound of the formula (I) where R⁶ = unsubstituted or substituted C₁-C₈-alkyl; and subsequently
**B)** preparing a compound of the formula (VII) by
6) reacting the compound of the formula (I) which has been obtained in step A) to give a compound of the formula (VII), 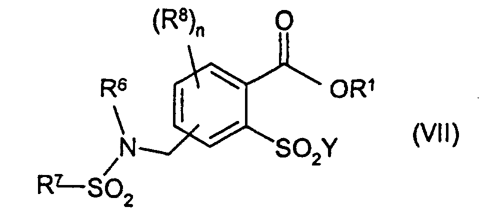 the compound of the formula (I) being reacted in the presence of an acid with a diazotizing agent and subsequently with an SO₂ source in the presence of a copper catalyst and an acid, where R¹, R⁶, R⁷, R⁸ and n in formula (VII) are as defined in formula (I) in claim 1 or 2 and Y = halogen; and subsequently
**C)** preparing the compound of the formula (XIII) by
**C1)** reacting the compound of the formula (VII) which has been obtained in step B6) with an amine of the formula (XII) in the presence of MOCN, wherein M is an ammonium ion or an alkali metal ion, to give a compound of the formula (XIII); or 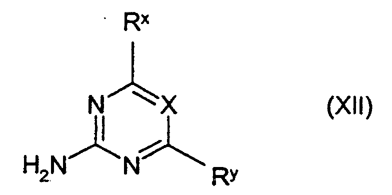
**C 2.1)** preparing a compound of the formula (VIII) by
7) reacting the compound of the formula (VII) which has been obtained in step B6) to give a compound of the formula (VIII), 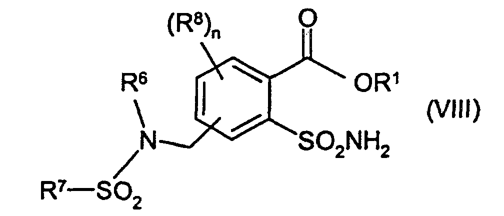 the compound of the formula (VIII) being subjected to aminolysis with ammonia in a suitable solvent, where R¹, R⁶, R⁷, R⁸ and n in formula (VIII) are as defined in formula (I) in claim 1 or 2;
and subsequently
**C2.2)** preparing the compound of the formula (XIII) by
9) reacting the compound of the formula (VIII) with a compound of the formula (IX) to give the compound of the formula (XIII); or 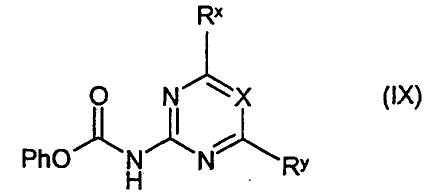
10) reacting the compound of the formula (VIII) with an isocyanate of the formula (X) to give the compound of the formula (XIII); or 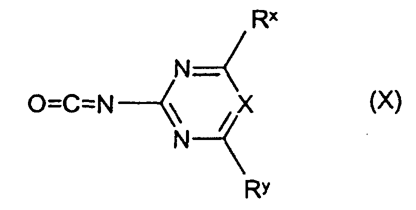
11) reacting the compound of the formula (VIII) with an alkyl isocyanate and phosgene to give a compound of the formula (XI), 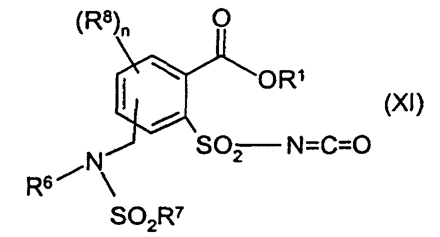 which is subsequently reacted with an amine of the formula (XII) to give the compound of the formula (XIII); or 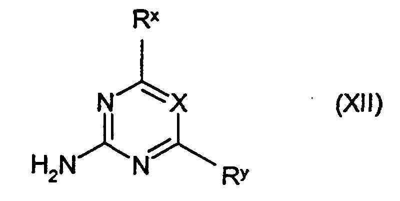
12) reacting the compound of the formula (VIII) with a carbonic acid derivative R-CO-OPh, in which Ph = unsubstituted or substituted phenyl and R = halogen or unsubstituted or substituted phenoxy to give a compound of the formula (XIV), 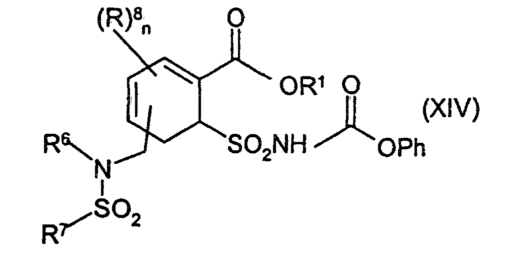 which is subsequently reacted with an amine of the formula (XII) to give the compound of the formula (XIII); 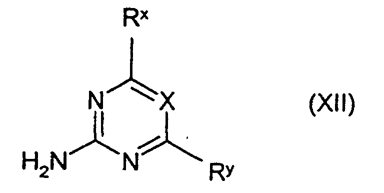
where R¹, R⁶, R⁷, R⁸ and n in formulae (XI) and (XIV) are as defined in formula (I) in claim 1 or 2, R^{x}, R^{y} and X in formulae (IX), (X) and (XII) are as defined in formula (XIII) of the present claim and Ph in formulae (IX) and (XIV) are unsubstituted or substituted phenyl.

6. A process for the preparation of a compound of the formula (XIII) as defined in claim 5, 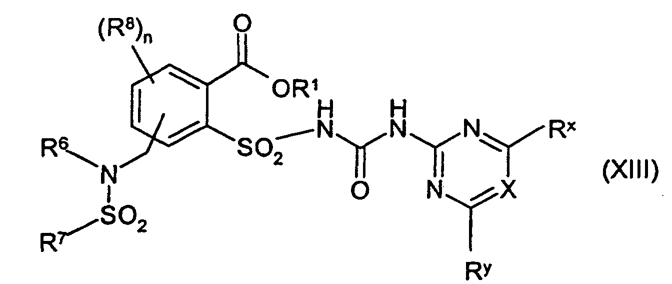 comprising steps B) and C1) or B), C2.1) and C2.2) as defined in claim 5.

7. A process for the preparation of a compound of the formula (VII) as defined in claim 5, 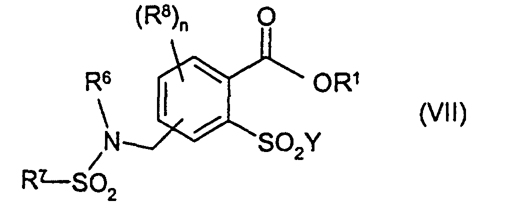 wherein
a compound of the formula (I) as defined in claim 1 or 2 and in which R², R³ = H is diazotized in the presence of an acid and subsequently reacted with an SO₂ source in the presence of a copper catalyst and an acid.

8. A process for the preparation of a compound of the formula (VIII) as defined in claim 5, 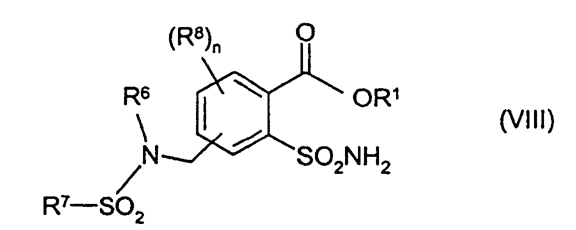 comprising the steps of
A) reacting a compound of the formula (I) as defined in claim 1 and in which R², R³ = H to give a compound of the formula (VII), 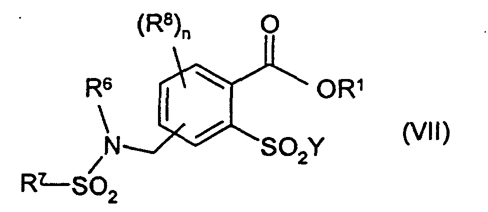 wherein the compound of the formula (I) is diazotized in the presence of an acid and subsequently reacted with an SO₂ source in the presence of a copper catalyst and of an acid,
and subsequently
B) reacting the compound of the formula (VII) to give a compound of the formula (VIII), 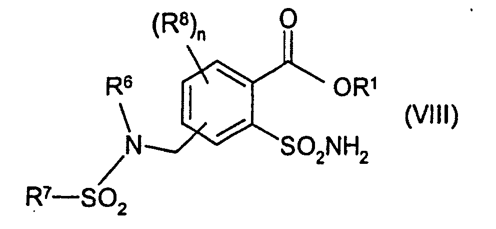 wherein the compound of the formula (VIII) is subjected to aminolysis in a suitable solvent with ammonia.

9. The use of a compound of the formula (II), (III), (IIIa), (IV), (V) or (Va) as defined in claim 4 for the preparation of a compound of the formula (I) as defined in claim 1 or 2.

10. The use of a compound of the formula (I) as defined in claim 1 or 2 for the preparation of a sulfonylurea.

11. The use of a compound of the formula (I) as defined in claim 1 or 2 for the preparation of a compound of the formula (VII), (VIII) or (XIII) as defined in claim 5.

12. A compound of the formula (VI) 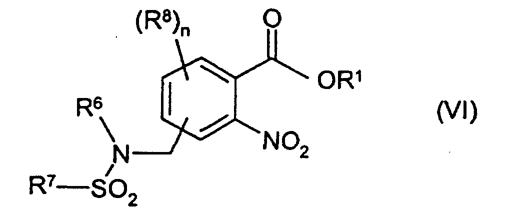 in which R¹, R⁶, R⁷, R⁸ and n are as defined in formula (I) as claimed in claim 1 or 2.

13. The use of a compound of the formula (VI) as defined in claim 12 for the preparation of a sulfonylurea.

14. The use of a compound of the formula (VI) as defined in claim 12 for the preparation of a compound of the formula (I) as defined in claim 1 or 2 or of a compound of the formula (VII), (VIII) or (XIII) as defined in claim 5.

15. A compound of the formula (Z) or (Za) 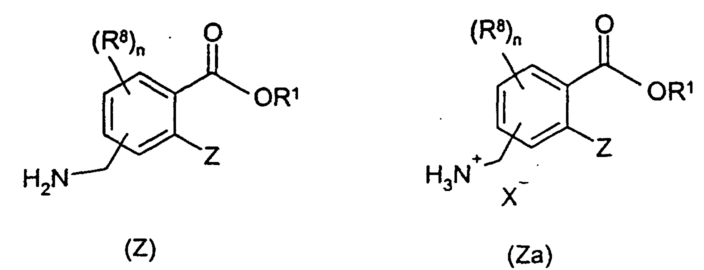 in which R¹, R⁸ and n are as defined in formula (I) as claimed in claim 1 or 2 and Z is NH₂ or NO₂.

16. The use of a compound of the formula (Z) or (Za) as defined in claim 15 for the preparation of a sulfonylurea.

17. The use of a compound of the formula (Z) or (Za) as defined in claim 15 for the preparation of a compound of the formula (I) as defined in claim 1 or 2 or of a compound of the formula (VII), (VIII) or (XIII) as defined in claim 5.

## Revendications

1. Composé de formule (I), 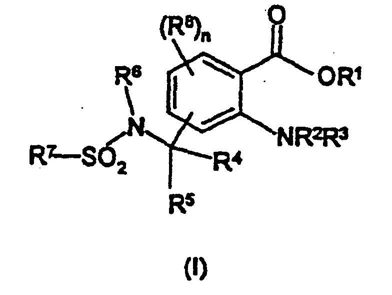 où
R¹ représente un atome d'hydrogène, un groupe alkyle en C₁-C₈, alcényle en C₃-C₈ ou alcynyle en C₃-C₈, les derniers trois restes sont non substitués ou substitués,
R², R³ indépendamment l'un de l'autre représentent un atome d'hydrogène ou un groupe acyle
R⁴, R⁵ représentent un atome d'hydrogène,
R⁶ représente un atome d'hydrogène ou un groupe alkyle en C₁-C₈, qui est non substitué, ou substitué,
R⁷ représente un groupe alkyle en C₁-C₈, alcényle en C₃-C₈, alcynyle en C₃-C₈, aryle en C₆-C₁₄ ou mono- ou di(alkyl en C₁-C₈)amino, qui sont non substitués ou substitués ou
R⁶ et R⁷ conjointement forment une chaîne de formule -(CH₂)ₘBₘ¹ qui est non substituée ou substituée, et où m = 2, 3 ou 4, m¹ = 0 ou 1 et B=CO ou SO₂,
R⁸ identiques ou différents représentent un groupe alkyle en C₁-C₄, alkoxy en C₁-C₄, (alkyl en C₁-C₄)carbonyle ou (alkoxy en C₁-C₄)carbonyle, qui sont non substitués ou substitués, ou R⁸ représente un atome d'halogène, un groupe NO₂ ou CN, et
n est égal à 0, 1, 2 ou 3.

2. Composé de formule (I) selon la revendication 1, où
R¹ représente un atome d'hydrogène ou un groupe alkyle en C₁-C₄, de préférence alkyle en C₁-C₄,
R², R³ représentent un atome d'hydrogène,
R⁴, R⁵ représentent un atome d'hydrogène,
R⁶ représente un atome d'hydrogène,
R⁷ représente un groupe alkyle en C₁-C₄, et
n est égal à 0.

3. Composé de formule (I) selon la revendication 1 ou 2, où le groupe -CR⁴R⁵-NR⁶-SO₂-R⁷ se trouve en position para par rapport au groupe -CO-OR¹.

4. Procédé pour la préparation d'un composé de formule (I), selon une ou plusieurs des revendications 1 à 3 comprenant les étapes où
1a) on fait réagir un composé de formule (II) 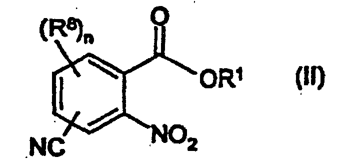 par hydrogénation catalytique en l'absence d'un acide pour obtenir un composé de formule (III) ou par hydrogénation catalytique en présence d'un acide H⁺X⁻, X⁻ étant un équivalent d'un anion acide, pour obtenir un composé de formule (IIIa), où X⁻ représente un équivalent d'un anion acide, 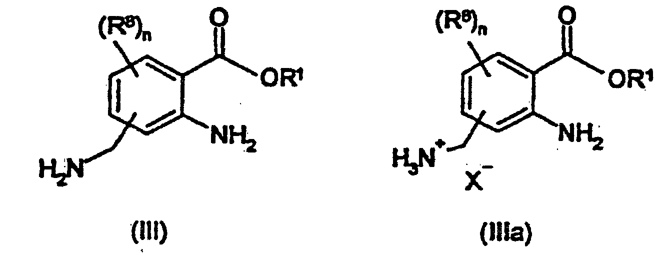 et ensuite,
1b) on fait réagir le composé de formule (III) ou (IIIa) sur un dérivé de l'acide sulfonique pour obtenir un composé de formule (I) dans lequel R², R³ und R⁶ = H ; ou
2a)
α) on fait réagir un composé de formule (II) 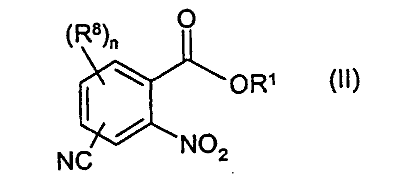 au moyen de procédés courants de réduction de composés nitrés pour obtenir un composé de formule (IV) 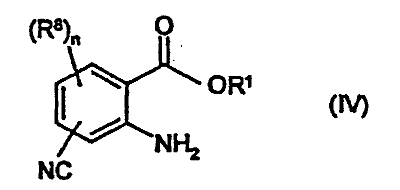 et ensuite
β) on fait réagir le composé de formule (IV) soit par hydrogénation catalytique au moyen de procédés courants de réduction de nitriles pour obtenir un composé de formule (III) ou (IIIa),
et ensuite
2b) on fait réagir le composé de formule (III) ou (IIIa) sur un dérivé de l'acide sulfonique pour donner un composé de formule (I) avec R², R³ et R⁶ = H ; ou
3a)
α) on fait réagir un composé de formule (II) 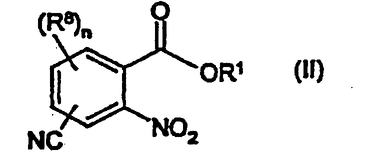 au moyen de procédés courants de réduction de nitriles pour obtenir un composé de formule (V) ou (Va), où X⁻ est défini comme à la formule (IIIa), 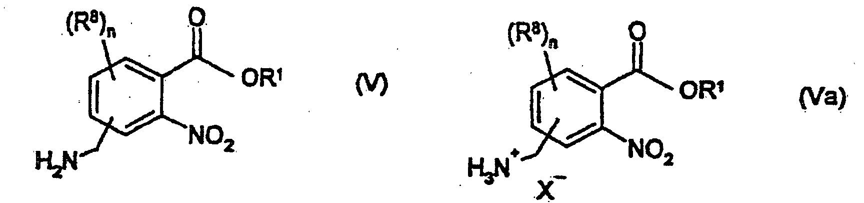 et finalement
β) on fait réagir le composé de formule (V) ou (Va) au moyen de procédés courants de réduction de composés nitrés ou par hydrogénation catalytique d'un composé de formule (III) ou (IIIa),
et ensuite
3b) on fait réagir le composé de formule (III) ou (IIIa) sur un dérivé de l'acide sulfonique en un composé de formule (I) avec R², R³ et R⁶ = H ; ou
4a)
α) un fait réagir un composé de formule (II) 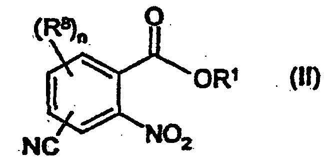 à l'aide de procédés courants de réduction de nitriles pour donner un composé de formule (V) ou (Va), où X⁻ est défini comme à la formule (IIIa), 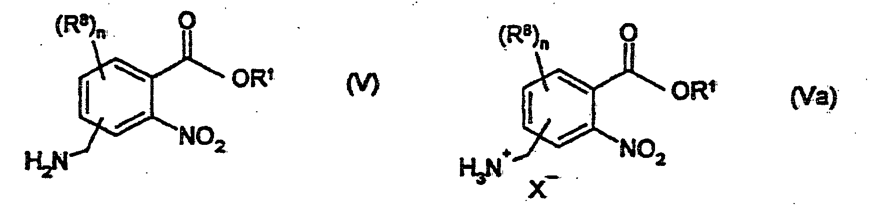
β) et finalement, on fait réagir le composé de formule (V) ou (Va) sur un dérivé de l'acide sulfonique pour obtenir un composé de formule (VI) 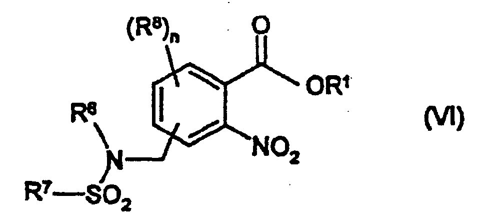
et finalement
4b) on fait réagir le composé de formule (VI) au moyen de procédés courants de réduction de composés nitrés ou par hydrogénation catalytique pour obtenir un composé de formule (I) avec R², R³ et R⁶ = H ;
R¹, R⁷, R⁸ et n dans les formules (II), (III), (IIIa), (IV), (V), (Va) et (VI) dont définis comme à la formule (I) dans la revendication 1 ou 2 ; et ensuite, facultativement,
5) on fait réagir le composé de formule (I) obtenu dans l'une des étapes 1)-4) sur un agent alkylant ou par amination réductrice pour obtenir un composé de formule (I) avec R⁶ = un groupe alkyle en C₁-C₈ non substitué ou substitué, ou à l'aide d'un agent acylant pour obtenir un composé de formule (I) avec R² et/ou R³ = acyle.

5. Procédé pour a préparation d'un composé de formule (XIII), où R¹, R⁶, R⁷, R⁸ et n sont définis comme à la formule (I) dans la revendication 1 ou 2, R^{x}, R^{y} indépendamment l'un de l'autre représentent un atome d'hydrogène, un atome d'halogène, un groupe alkyle en C₁-C₄, alkoxy en C₁-C₄, (alkyl en C₁-C₄)thio, chacun des 3 derniers restes cités est non substitué ou substitué par un ou plusieurs restes pris parmi les restes halogène, alkoxy en C₁-C₄ et (alkyl en C₁-C₄)thio, ou représentent mono- ou di (alkyl en C₁-C₄)amino, alcényle en C₂-C₆, alcynyle en C₂-C₆, alcényloxy en C₃-C₆ ou alcynyloxy en C₃-C₆, et
X représente un groupe CH ou N, 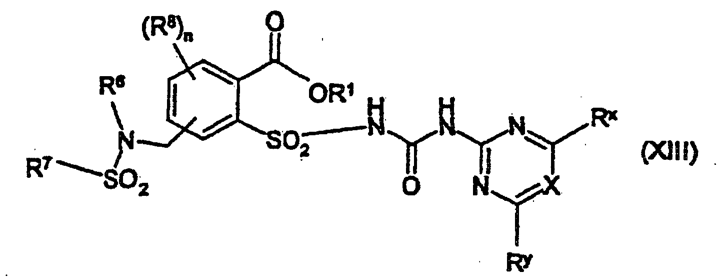
comprenant les étapes de
A) on prépare un composé de formule (I) comme défini à la revendication 1 ou 2 et R² et R³ = H, où
1a) on fait réagir un composé de formule (II) 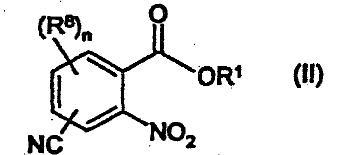 par hydrogénation catalytique en l'absence d'un acide pour obtenir un composé de formule (III) ou par hydrogénation catalytique en présence d'un acide H⁺X⁻, X⁻ est un équivalent d'un anion acide, pour donner un composé de formule (IIIa), où X⁻ est un équivalent d'un anion acide, 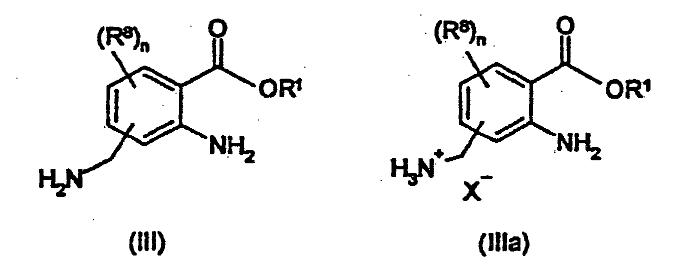 et ensuite
1b) on fait réagir le composé de formule (III) ou (IIIa) sur un dérivé de l'acide sulfonique pour donner un composé de formule (I) avec R², R³ et R⁶ = H ; ou
2a)
α) on fait réagir un composé de formule (II) 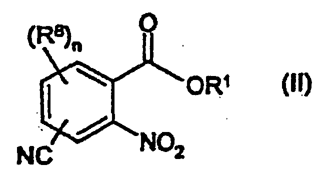 à l'aide de procédés courants de réduction de composés nitrés pour obtenir un composé de formule (IV), 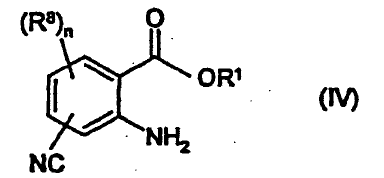 et ensuite
β) on fait réagir le composé de formule (IV) soit par hydrogénation catalytique, soit par des procédés courants de réduction de nitriles, pour obtenir un composé de formule (III) ou (IIIa),
et ensuite
2b) on fait réagir le composé de formule (III) ou (IIIa) sur un dérivé de l'acide sulfonique pour donner un composé de formule (I) avec R², R³ et R⁶ = H ; ou
3a)
α) on fait réagir un composé de formule (II) 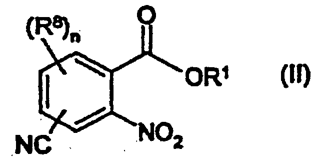 au moyen de procédés courants de réduction de nitriles pour obtenir un composé de formule (V) ou (Va), où X⁻ est défini comme à la formule (IIIa), 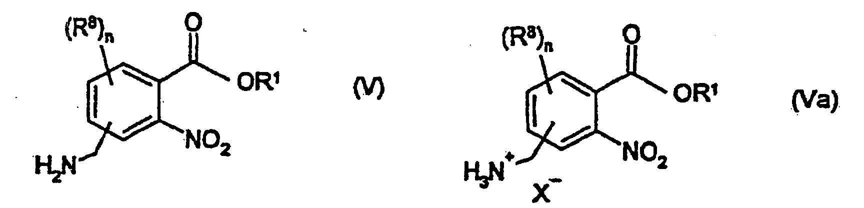 et ensuite
β) on fait réagir le composé de formule (V) ou (Va) à l'aide de procédés courants de réduction de composés nitrés ou par hydrogénation catalytique pour obtenir un composé de formule (III) ou (IIIa),
et ensuite
3b) on fait réagir le composé de formule (III) ou (IIIa) sur un dérivé de l'acide sulfonique pour obtenir un composé de formule (I) R², R³ et R⁶ = H ; ou
4a)
α) on fait réagir un composé de formule (II) 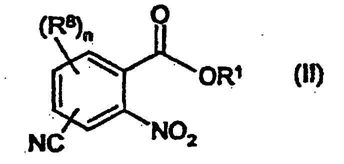 à l'aide de procédés courants de réduction de nitriles pour obtenir un composé de formule (V) ou (Va), où X⁻ est défini comme à la formule (IIIa), 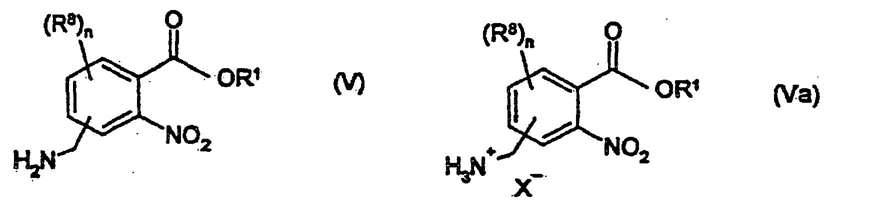
β) et ensuite on fait réagir un composé de formule (V) ou (Va) sur un dérivé de l'acide sulfonique pour obtenir un composé de formule (VI), 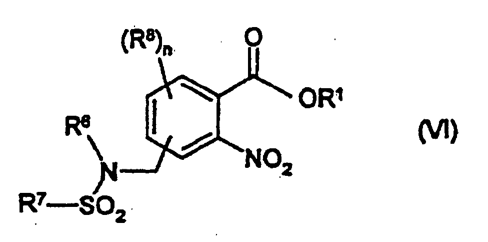
et ensuite
4b) on fait réagir le composé de formule (VI) à l'aide de procédés courants de réduction de composés nitrés ou par hydrogénation catalytique pour obtenir un composé de formule (I) avec R², R³ et R⁶ = H ;
R¹, R⁷, R⁸ et n dans les formules (II), (III), (IIIa), (IV), (V), (Va) et (VI) sont définis comme à la formule (I) dans la revendication 1 ou 2 ; et ensuite, facultativement,
5) on fait réagir le composé de formule (I) obtenu dans l'une des étapes A1)-A4) sur un agent alkylant ou par amination réductrice pour obtenir un composé de formule (I) avec R⁶ = un groupe alkyle en C₁-C₈ non substitué ou substitué ; et ensuite
B) on prépare un composé de formule (VII),
6) en faisant réagir le composé de formule (I) obtenu à l'étape A) pour obtenir un composé de formule (VII), 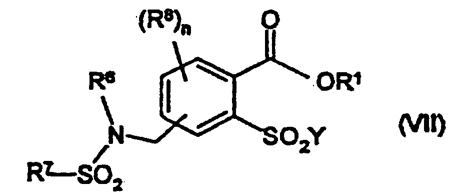 par diazotation du composé de formule (I) en présence d'un acide à l'aide d'un agent de diazotation et ensuite par transformation à l'aide d'une source de SO₂ en présence d'un catalyseur au cuivre et d'un acide, où R¹, R⁶, R⁷, R⁸ et n à la formule (VII) sont définis comme à la revendication 1 ou 2 et Y = halogène ; et ensuite
C) on prépare le composé de formule (XIII),
C1) en faisant réagir le composé de formule (VII) obtenu à l'étape B6), en présence de MOCN, où M est un ion ammonium ou un ion métal alcalin, sur une amine de formule (XII) pour obtenir un composé de formule (XIII) ; ou 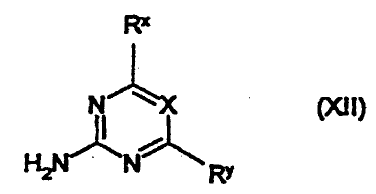
C2.1) on prépare un composé de formule (VIII),
7) en faisant réagir le composé de formule (VII) obtenu à l'étape B6) pour obtenir un composé de formule (VIII), 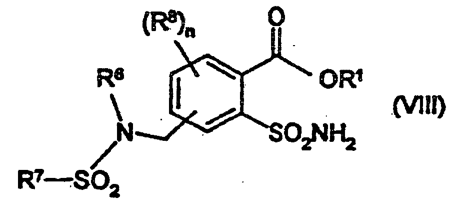 par aminolyse du composé de formule (VIII) dans un solvant approprié par l'ammoniac, R¹, R⁶, R⁷, R⁸ et n à la formule (VIII) sont définis comme à la formule (I) dans la revendication 1 ou 2 ;
et ensuite
C2.2) on prépare le composé de formule (XIII),
9) en faisant réagir le composé de formule (VIII) sur le composé de formule (IX) pour obtenir le composé de formule (XIII) ; ou 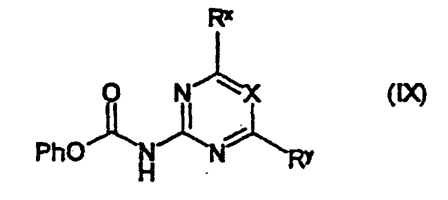
10) en faisant réagir le composé de formule (VIII) sur un isocyanate de formule (X) pour donner le composé de formule (XIII) ; ou 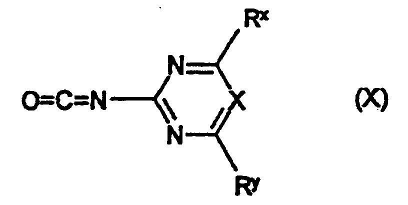
11) en faisant réagir le composé de formule (VIII) sur un isocyanate alcalin et le phosgène pour obtenir le composé de formule (XI), 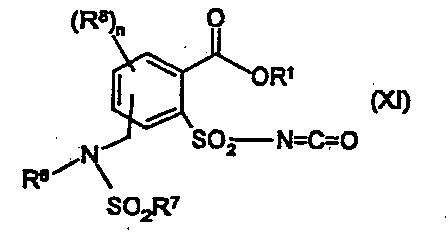 que l'on fait réagir ensuite sur une amine de formule (XII) en le composé de formule (XIII) ; ou 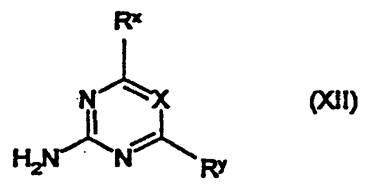
12) en faisant réagir le composé de formule (VIII) sur un dérivé de l'acide carbonique R-CO-OPh, où Ph = phényle non substitué ou substitué et R = halogène ou phénoxy non substitué ou substitué, pour obtenir un composé de formule (XIV), 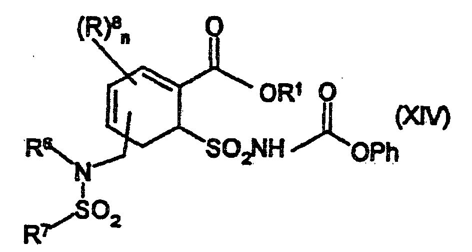 qu'on fait réagir ensuite sur une amine de formule (XII) pour obtenir le composé de formule (XIII) ; 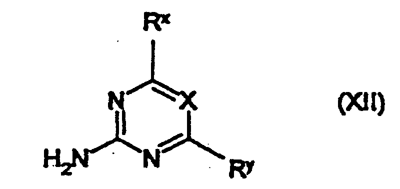
où R¹, R⁶, R⁷, R⁸ et n dans les formules (XI) et (XIV) sont définis comme à la formule (I) dans la revendication 1 ou 2, R^{x}, R^{y} et X aux formules (IX), (X) et (XII) sont définis comme à la formule (XIII) de cette revendication et Ph dans les formules (IX) et (XIV) représente un groupe phényle non substitué ou substitué.

6. Procédé pour la préparation d'un composé de formule (XIII) comme défini à la revendication 5, 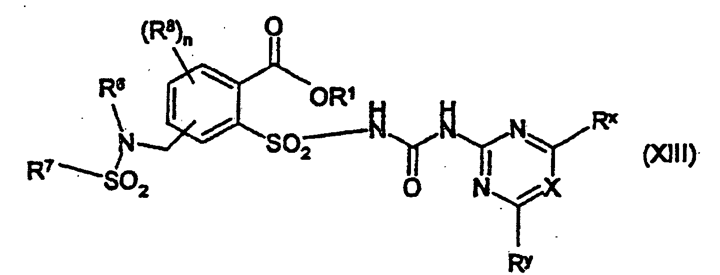 comprenant les étapes B) et C1) ou B), C2.1) et C2.2) définis comme à la revendication 5.

7. Procédé pour la préparation d'un composé de formule (VII) défini comme à la revendication 5, 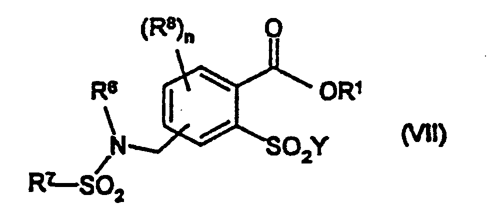 où
on diazote un composé de formule (I) comme défini à la revendication 1 ou 2 et où R², R³ = H, en présence d'un acide et ensuite on fait réagir sur une source de SO₂ en présence d'un catalyseur au cuivre et d'un acide.

8. Procédé pour la préparation d'un composé de formule (VIII) défini comme à la revendication 5, 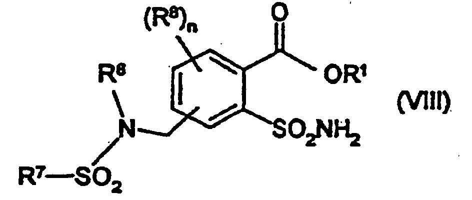 comprenant les étapes, où
A) on fait réagir un composé de formule (I) comme défini à la revendication 1 et où R², R³ = H, pour obtenir un composé de formule (VII), 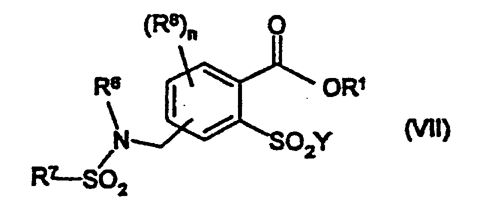 en diazotant le composé de formule (I) en présence d'un acide et ensuite on fait réagir sur une source de SO₂ en présence d'un catalyseur au cuivre et d'un acide,
et ensuite
B) on fait réagir le composé de formule (VII) en un composé de formule (VIII), 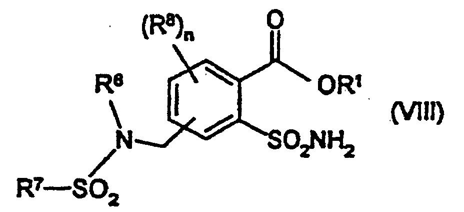 par aminolyse par l'ammoniaque du composé de formule (VIII) dans un solvant approprié.

9. Utilisation d'un composé de formule (II), (III), (IIIa), (IV), (V) ou (va) comme défini à la revendication 4, pour la préparation d'un composé de formule (I) comme défini à la revendication 1 ou 2.

10. Utilisation d'un composé de formule (I) comme défini à la revendication 1 ou 2, pour la préparation d'une sulfonylurée.

11. Utilisation d'un composé de formule (I) comme défini à la revendication 1 ou 2, pour la préparation d'un composé de formule (VII), (VIII) ou (XIII) comme défini à la revendication 5.

12. Composé de formule (VI) 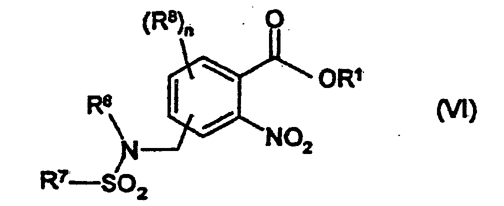 où R¹, R⁶, R⁷, R⁸ et n sont définis comme à la formule (I) selon la revendication 1 ou 2.

13. Utilisation d'un composé de formule (VI) comme défini à la revendication 12, pour la préparation d'une sulfonylurée.

14. Utilisation d'un composé de formule (VI) comme défini à la revendication 12, pour la préparation d'un composé de formule (I) comme défini à la revendication 1 ou 2 ou d'un composé de formule (VII), (VIII) ou (XIII) comme défini à la revendication 5.

15. Composé de formule (Z) ou (Za) 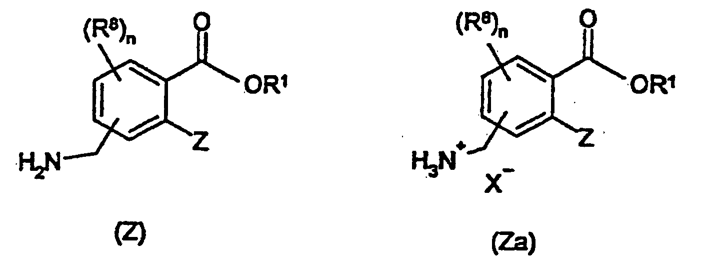 où R¹, R⁸ et n sont définis comme à la formule (I) selon la revendication 1 ou 2 et Z est égal à NH₂ ou NO₂.

16. Utilisation d'un composé de formule (Z) ou (Za) comme défini à la revendication 15, pour la préparation d'une sulfonylurée.

17. Utilisation d'un composé de formule (Z) ou (Za) comme défini à la revendication 15, pour la préparation d'un composé de formule (I) comme défini à la revendication 1 ou 2 ou d'un composé de formule (VII), (VIII) ou (XIII) comme défini à la revendication 5.
